(19) Europäisches Patentamt
European Patent Office
Office européen des brevets

(11) EP 2 785 745 B1

(12) **FASCICULE DE BREVET EUROPEEN**

(45) Date de publication et mention de la délivrance du brevet:
**21.02.2024 Bulletin 2024/08**

(21) Numéro de dépôt: **12813409.5**

(22) Date de dépôt: **03.12.2012**

(51) Classification Internationale des Brevets (IPC):
***C08B 15/00*** (2006.01) ***C08B 37/00*** (2006.01)
***C08L 1/28*** (2006.01) ***C08L 5/00*** (2006.01)
***C08L 5/08*** (2006.01) ***A61K 31/717*** (2006.01)
***A61K 31/722*** (2006.01) ***A61K 31/728*** (2006.01)
***A61K 31/738*** (2006.01)

(52) Classification Coopérative des Brevets (CPC):
**C08B 15/005; A61K 8/735; A61K 31/715; A61K 31/717; A61K 31/722; A61K 31/728; A61K 31/738; A61Q 19/08; C07H 15/12; C08B 37/00; C08B 37/0006; C08B 37/003; C08B 37/0072; C08L 1/286; C08L 5/00;** (Cont.)

(86) Numéro de dépôt international:
**PCT/FR2012/052781**

(87) Numéro de publication internationale:
**WO 2013/079889 (06.06.2013 Gazette 2013/23)**

(54) **PROCÉDÉ DE SUBSTITUTION ET RÉTICULATION SIMULTANÉES D'UN POLYSACCHARIDE VIA SES FONCTIONS HYDROXYLES**

VERFAHREN ZUR GLEICHZEITIGEN SUBSTITUTION UND VERNETZUNG EINES POLYSACCHARIDS MITTELS DER HYDROXY-FUNKTIONEN DAVON

METHOD FOR SIMULTANEOUSLY SUBSTITUTING AND CROSS-LINKING A POLYSACCHARIDE BY MEANS OF THE HYDROXYL FUNCTIONS THEREOF

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **02.12.2011 US 201161566258 P**
**02.12.2011 FR 1161125**

(43) Date de publication de la demande:
**08.10.2014 Bulletin 2014/41**

(73) Titulaire: **Laboratoires Vivacy**
**75116 Paris (FR)**

(72) Inventeurs:
- **BON BETEMPS, Jérémie**
**F-73410 Albens (FR)**
- **PIRON, Estelle**
**F-73130 Saint Etienne De Cuines (FR)**

(74) Mandataire: **Tripoz, Inès**
**Cabinet Tripoz**
**Le Pôle Sud**
**22 rue Seguin**
**69002 Lyon (FR)**

(56) Documents cités:
**EP-A1- 1 942 117**
**WO-A2-2011/148116**
**US-A- 4 175 183**
**US-A- 4 321 367**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

(52) Classification Coopérative des Brevets (CPC):
(Cont.)
**C08L 5/08;** A61K 9/0019; A61K 47/36;
A61K 2800/91

## Description

La présente invention concerne de nouveaux polysaccharides biocompatibles réticulés et substitués, caractérisés par des propriétés rhéologiques améliorées et présentant éventuellement des propriétés d'intérêt apportées par les substituants comme par exemple une action hydratante ou lipophilisante, par rapport à un polysaccharide réticulé classique et pouvant être utilisés comme biomatériaux notamment dans le domaine de la chirurgie de comblement, la réparation tissulaire ou comme matériau ou fluides articulaires.

On connait de l'art antérieur de nombreux polysaccharides substitués et réticulés, mais aucun des procédés de l'art antérieur n'a permis d'obtenir des polysaccharides présentant les propriétés rhéologiques améliorées de façon synergique selon la présente invention.

On connait de EP 0 256 116, au nom de FIDIA, des composés réticulés et substitués via des fonctions esters sur les fonctions -COOH du polysaccharide. Les chaines d'acide hyaluronique sont en effet réticulées via des ponts ester formés par la réaction intra- ou intermoléculaire entre des polyalcools aliphatiques et les fonctions acide carboxylique du polysaccharide. La substitution se fait également via les fonctions ester du polysaccharide par le greffage de petites molécules porteuses de fonctions hydroxyles comme l'éthanol ou l'alcool benzylique par exemple.

Dans EP 0 341 745, au nom de FIDIA également, le polymère est dit « auto-réticulé » dans le sens que les fonctions esters sont formées entre les fonctions carboxyles du polysaccharide et les fonctions hydroxyles d'une même chaine ou d'une autre chaine, sans réticulant. Le brevet divulgue également des polymères réticulés et substitués. Les substituants sont ici aussi des chaines alcool telles que de l'éthanol ou l'alcool benzylique et sont greffés sur le polysaccharide via les fonctions carboxyles de celui-ci.

La demande de brevet WO 99/43728, au nom de FIDIA, fait état d'acides hyaluroniques sulfatés en N-position ou O-position. Ces polysaccharides, qui peuvent comme dans les demandes de brevet citées ci-dessus être autoréticulés ou réticulés via des ponts ester (EP 0 256 116 et EP 0 341 745), sont ensuite greffés avec des polyuréthanes. On obtient ainsi une matrice complexe de différents polymères co-réticulés. Cependant, ces produits sont également réticulés via des fonctions ester et nécessitent de plus la mise en oeuvre d'au moins trois étapes de synthèse dans le cas d'une matrice de polysaccharide O- ou N-sulfatés et de polyuréthane. Ils ne présentent ainsi pas une solution satisfaisante.

Le brevet EP 0 749 982, au nom de HERCULES, enseigne la préparation de polymères substitués par des composés phénols encombrés, aux propriétés antioxydantes qui peuvent éventuellement être réticulés. La réticulation est réalisée par des moyens classiques de l'art antérieur à l'aide d'époxydes polyfonctionnels ou halohydrines correspondantes (US 4,716,224, US 4,863,907, EP 0 507 604 A2, US 4,716,154, US 4,772,419, US 4,957,744), d'alcools polyhydriques (US 4,582,865, US 4,605,691), de divinylsulfone (US 5,128,326, US 4,582,865) et d'aldéhydes (US 4,713,448). Selon le mode de réalisation préféré, le polymère est réticulé par réaction avec des acides carboxyliques ou des polyanhydrides d'acide, pour conduire à nouveau à la formation d'esters. Les composés décrits dans l'art antérieur ont ainsi une forte densité en fonctions ester dans la plupart des cas.

On connait également de l'art antérieur des polymères co-réticulés, par exemple ceux décrits dans la demande de brevet WO 2005/012364, au nom de ANTEIS, qui présentent une rémanence améliorée, par la formation d'une matrice par co-réticulation d'un ou plusieurs polymères. Ces polysaccharides co-réticulés peuvent en outre être substitués par des polymères de masse moléculaire moyenne basse ou des petites molécules non-polymériques. Ces polymères sont d'une manière générale de l'acide hyaluronique co-réticulé avec de la cellulose, composé sur lequel seront ensuite greffés, via l'agent réticulant, par des polymères de petite taille comme de l'héparine ou de l'acide hyaluronique porteur d'esters benzyliques (Mw < 50 000 kDa). Dans certains cas, des antioxydants comme la vitamine C sont greffés également via le réticulant.

Le brevet US 4,605,691, au nom de Balazs (BIOMATRIX), enseigne également un procédé de co-réticulation (de formation de polymères co-réticulés). Il s'agit cette fois de co-réticulation d'acide hyaluronique avec du collagène, de la cellulose, de l'héparine ou de l'acide carminique.

Le point commun des procédés décrits dans l'art antérieur est que les substitutions ou greffages qui conduisent à la fonctionalisation se font via l'agent réticulant. Par rapport à une réticulation seule, cela se traduit par une compétition entre réaction de réticulation et fonctionnalisation via l'agent de réticulation.

L'homme de l'art devra ainsi toujours veiller à bien adapter la quantité de réticulant introduite pour que la fonctionnalisation ne limite pas la réticulation, ce qui entraînerait une modification des propriétés finales du gel en fonction des cinétiques respectives des réactions.

En effet, si la réticulation a lieu avant l'introduction de l'agent fonctionnel, il faudra veiller d'une part à ce que l'agent fonctionnel s'introduise de façon homogène dans le réseau polymérique réticulé, et d'autre part à ce qu'il reste suffisamment d'agent réticulant pour fonctionnaliser le polymère. Si tout est consommé, il faut alors rajouter de l'agent réticulant, on s'expose alors à une surréticulation éventuelle. Dans certains cas, l'homme de l'art sera même obligé de substituer avant de réticuler pour limiter les effets dits de « compétition ».

L'homme de l'art sera ainsi toujours confronté à un choix : substituer avant ou après avoir réticulé le polymère

et adapter les conditions réactionnelles en tenant compte des vitesses de réactions polymère/agent réticulant et agent fonctionnalisant/agent réticulant. Ce choix devient ainsi une étape cruciale et délicate du procédé.

On connait également des celluloses hydrosolubles telles que les sulfoalkylcelluloses substituées par des sulfoalkyles et des éthers. Le but recherché est d'obtenir une grande fonctionnalisation pour obtenir des sulfoalkylcelluloses solubles dans l'eau, voir par exemple US 4,990,609 au nom de WOLFF WALSRODE. Pour ce type de fonctionnalisation, les sultones sont souvent utilisées pour leur grande réactivité qui permet un fort taux de fonctionnalisation et améliore la solubilité du produit final de par la forte présence de fonctions sulfonates. Mais si on souhaite réticuler dans un deuxième temps, les degrés élevés de substitution peuvent nuire à la bonne réticulation du polymère. De plus, les conditions de fonctionnalisation sont souvent trop drastiques pour que le polysaccharide ne soit pas dégradé au point de diminuer de façon irrémédiable ses propriétés rhéologiques, voir par exemple les composés décrits dans US 3,046,272.

Il est en effet connu que les polysaccharides, par exemple l'acide hyaluronique, résistent relativement mal aux conditions alkalines et il est connu que lors de la réticulation ou la déacétyation de l'acide hyaluronique dans la soude, une dégradation se produit (Simkovic et al., Carbohydrate Polymers, 41, 2000, 9-14), or les réactions décrites dans l'art antérieur sont souvent longues et/ou dans des conditions de pH qui entrainent une dégradation.

Dans les conditions alcalines très concentrées et aux températures élevées auxquelles sont soumis les polysaccharides dans des procédés tels que ceux décrits dans US 4,321,367 ou dans US 4,175,183, il a été démontré, voir les exemples comparatifs ci-après, qu'une dégradation du polysaccharide se produit.

Ainsi, dans les procédés décrits dans l'art antérieur, bien que relativement faciles à mettre en oeuvre, les procédés pour réaliser un polymère réticulé et substitué sont bien souvent longs et nécessitent plusieurs étapes de par l'ajout successif des différents ingrédients pour éviter une compétition entre les différents polymères et greffons qui vont se fixer sur le polysaccharide via l'agent réticulant. De plus, du fait des temps de réaction et des conditions réactionnelles, le polymère résultant peut avoir des propriétés rhéologiques dégradées.

La présente invention permet de résoudre l'ensemble des inconvénients des procédés de l'art antérieur et permet en outre d'obtenir des polysaccharides dont les propriétés rhéologiques sont améliorées de façon synergique.

Elle concerne un procédé de préparation de polysaccharides réticulés et substitués. Ce procédé est caractérisé principalement en ce que les réactions de réticulation et de substitution sont mises en oeuvre de manière simultanée, dans les mêmes conditions expérimentales et sur les mêmes sites réactionnels, les fonctions hydroxyles du polysaccharide, et ce, sans qu'il y ait de compétition entre les différentes espèces mises en jeu, les substitutions n'étant pas effectuées via l'agent réticulant. Les taux de substitution et de réticulation obtenus sont donc comparables à ceux obtenus par des réactions réalisées de manière séquentielle et les propriétés rhéologiques des polysaccharides sont améliorées.

La présente invention concerne également un polysaccharide réticulé et substitué obtenu par le procédé selon l'invention, dont la rhéologie, et en particulier la viscoélasticité, est accrue par rapport non seulement au polysaccharide simplement substitué, mais également par rapport au polysaccharide uniquement réticulé, et par rapport au polysaccharide substitué puis réticulé.

Les substituants peuvent en outre apporter des propriétés d'intérêt, par exemple biologiques, au polysaccharide selon l'invention, dont les propriétés rhéologiques sont améliorées. L'effet synergique est d'autant plus surprenant qu'il est conservé lors de la stérilisation du polysaccharide substitué et réticulé.

La présente invention permet ainsi de combiner les avantages relatifs à la substitution et ceux relatifs à la réticulation sans modifier les caractéristiques individuelles de chacune de ces modifications prises séparément et surtout sans dégrader les propriétés rhéologiques puisqu'elles sont améliorées de façon synergique.

L'invention concerne un procédé de substitution et réticulation simultanées d'un polysaccharide via ses fonctions hydroxyles, en phase aqueuse, comprenant les étapes suivantes :

- on dispose d'un polysaccharide en milieu aqueux, choisi dans le groupe constitué par l'acide hyaluronique ou l'un de ses sels, le chitosane et leurs dérivés,
- on le met en présence d'au moins un précurseur d'un substituant, choisi dans le groupe constitué des molécules comprenant une seule fonction réactive choisie dans le groupe consitué des fonctions vinyle substituée ou non, époxyde substituée ou non, allyle substituée ou non, cétone, aldéhyde, thiocyanate, halide, isocyanate, halosilicium, nitrile, sultone,
- on le met en presence d'un agent de réticulation,
- on obtient et on isole le polysaccharide substitué et réticulé,

caractérisé en ce qu'il est mis en oeuvre en présence d'un catalyseur, base ou acide, dont la concentration est comprise entre $3{,}16.10^{-7}$ et 0,32 mol/L, et à une température est inférieure à 60°C.

On entend par « via ses fonctions hydroxyles », le fait que les substitutions et les réticulations sont effectuées sur les -OH portés par les polysaccharides.

Le procédé peut être également caractérisé par un ratio cataliseur réactionnel ou RCR.

Ce ratio catalyseur réactionnel (RCR) est défini comme étant :

$$RCR = \frac{\text{(Nombre de mole de fonctions réactives du catalyseur introduites dans le milieu réactionnel)}}{\text{(Nombre de moles de motif disaccharidique introduites dans le milieu réactionnel)}}$$

Dans un mode de réalisation, le procédé selon l'invention est caractérisé en ce que le ratio catalyseur réactionnel est compris entre 0,02:1 et 3:1.

Dans un mode de réalisation, le procédé selon l'invention est caractérisé en ce que le ratio catalyseur réactionnel est compris entre 0,2:1 et 3:1.

Dans un mode de réalisation, le procédé selon l'invention est caractérisé en ce que le ratio catalyseur réactionnel est compris entre 0,3:1 et 3:1.

Dans un mode de réalisation, le procédé selon l'invention est caractérisé en ce que le ratio catalyseur réactionnel est compris entre 0,5:1 et 2:1.

Dans un mode de réalisation, le procédé selon l'invention est caractérisé en ce que le ratio catalyseur réactionnel est compris entre 0,7:1 et 1,5:1.

Dans un mode de réalisation, le procédé selon l'invention est caractérisé en ce que le ratio catalyseur réactionnel est 1,75:1.

Dans un mode de réalisation, le procédé selon l'invention est caractérisé en ce que le ratio catalyseur réactionnel est 1:1.

Dans un mode de réalisation, le procédé selon l'invention est caractérisé en ce que le ratio catalyseur réactionnel est 0,8:1.

Dans un mode de réalisation, le procédé selon l'invention est caractérisé en ce que le ratio catalyseur réactionnel est 0,06:1.

Dans un mode de réalisation, le procédé selon l'invention est caractérisé en ce que le catalyseur est une base.

Dans ce mode de réalisation, la fonction réactive du catalyseur est l'ion $HO^-$.

En phase aqueuse, on a $pH = 14 + \log([HO^-])$ et $[HO^-] = 10^{-(14-pH)}$,

Dans un mode de réalisation, le procédé selon l'invention est caractérisé en ce que la concentration en catalyseur $HO^-$ est comprise entre $10^{-6}$ mol/L et 0,32 mol/L, tel que $10^{-6}$ mol/L $\leq [HO^-] \leq$ 0,32 mol/L.

Dans un mode de réalisation, le procédé selon l'invention est caractérisé en ce que la concentration en catalyseur $HO^-$ est comprise entre $3{,}16.10^{-4}$ mol/L et $3{,}16.10^{-2}$ mol/L, tel que $3{,}16.10^{-4}$ mol/L $\leq [HO^-] \leq 3{,}16.10^{-2}$ mol/L.

Dans un mode de réalisation, le procédé selon l'invention est caractérisé en ce que le catalyseur est une base inorganique.

Dans un mode de réalisation, le procédé selon l'invention est caractérisé en ce que la base inorganique est choisie dans le groupe comprenant la soude (hydroxyde de sodium) ou la potasse (hydroxyde de potassium).

Dans un mode de réalisation, le procédé selon l'invention est caractérisé en ce que la concentration massique de la base inorganique est comprise entre $1{,}2.10^{-5}$% et 1,3%.

Dans un mode de réalisation, le procédé selon l'invention est caractérisé en ce que la concentration massique de la base inorganique est comprise entre 0,25 et 1,15%.

Dans un mode de réalisation, le procédé selon l'invention est caractérisé en ce que la concentration massique de la base inorganique est comprise entre 0,25 et 1,1%.

Dans un mode de réalisation, le procédé selon l'invention est caractérisé en ce que la concentration massique de la base inorganique est 1%.

Dans un mode de réalisation, le procédé selon l'invention est caractérisé en ce que la concentration massique de la base inorganique est 0,5%.

Dans un mode de réalisation, le procédé selon l'invention est caractérisé en ce que le catalyseur est une base organique.

Dans un mode de réalisation, le procédé selon l'invention est caractérisé en ce que la base organique est la pyridine.

Dans un mode de réalisation, le procédé selon l'invention est caractérisé en ce que le pH du milieu réactionnel aqueux est basique.

Dans un mode de réalisation, le procédé selon l'invention est caractérisé en ce que le pH du milieu réactionnel aqueux est compris dans un intervalle de 8 à 13,5.

Dans un mode de réalisation, le procédé selon l'invention est caractérisé en ce que le pH du milieu réactionnel aqueux est compris dans un intervalle de 10,5 à 12,5.

Dans un mode de réalisation, le procédé selon l'invention est caractérisé en ce que le catalyseur est un acide.

Dans ce mode de réalisation, la fonction réactive du catalyseur est l'ion $H_3O^+$.

Dans un mode de réalisation, le procédé selon l'invention est caractérisé en ce que la concentration en catalyseur $H_3O^+$ est comprise entre $3{,}16.10^{-7}$mol/L et 0,01 mol/L, tel que $3{,}16.10^{-7}$mol/L $\leq [H_3O^+] \leq$ 0,01 mol/L.

Dans un mode de réalisation, le procédé selon l'invention est caractérisé en ce que la concentration en catalyseur $H_3O^+$ est comprise entre $10^{-6}$ mol/L et $3{,}16.10^{-5}$ mol/L, tel que $10^{-6}$ mol/L $\leq [H_3O^+] \leq 3{,}16.10^{-5}$ mol/L.

En phase aqueuse, on a $pH = -\log([H_3O^+])$ et $[H_3O^+] = 10^{-pH}$.

Dans un mode de réalisation, le procédé selon l'invention est caractérisé en ce que l'acide est un acide inorganique.

Dans un mode de réalisation, le procédé selon l'invention est caractérisé en ce que l'acide inorganique est l'acide chlorhydrique.

Dans un mode de réalisation, le procédé selon l'invention est caractérisé en ce que la concentration massique de l'acide inorganique est comprise entre $1{,}14.10^{-5}$ et 1,15 %.

Dans un mode de réalisation, le procédé selon l'invention est caractérisé en ce que la concentration massique de l'acide inorganique est comprise entre 0,05 et 1%.

Dans un mode de réalisation, le procédé selon l'invention est caractérisé en ce que la concentration massique de l'acide inorganique est comprise entre 0,05 et 0,36%.

Dans un mode de réalisation, le procédé selon l'invention est caractérisé en ce que le catalyseur est un acide organique.

Dans un mode de réalisation, l'acide organique est choisi dans le grroupe comprenant l'acide glutamique ou l'acide acétique.

Dans un mode de réalisation, le procédé selon l'invention est caractérisé en ce que la concentration massique de l'acide organique est comprise entre 0,25 et 2%.

Dans un mode de réalisation, le procédé selon l'invention est caractérisé en ce que la concentration massique de l'acide organique est comprise entre 0,25 et 1,1%.

Dans un mode de réalisation, le procédé selon l'invention est caractérisé en ce que la concentration massique de l'acide organique est 1%.

Dans un mode de réalisation, le procédé selon l'invention est caractérisé en ce que le pH du milieu réactionnel aqueux est acide.

Dans un mode de réalisation, le procédé selon l'invention est caractérisé en ce que le pH du milieu réactionnel aqueux est compris dans un intervalle de 2 à 6,5.

Dans un mode de réalisation, le procédé selon l'invention est caractérisé en ce que le pH du milieu réactionnel aqueux est compris dans un intervalle de 4,5 à 6.

Le catalyseur, base ou acide, est soluble en milieux aqueux.

Dans un mode de réalisation, le procédé selon l'invention est caractérisé en ce que le polysaccharide est choisi dans le groupe constitué par l'acide hyaluronique ou l'un de ses sels, le chitosane et leurs dérivés.

Dans un mode de réalisation, le polysaccharide est l'acide hyaluronique.

Dans un mode de réalisation, le polysaccharide est le hyaluronate de sodium.

Dans un mode de réaliastion, le polysaccharide est le chitosane.

Dans un mode de réalisation, le chitosane est partiellement déacétylé.

Dans un mode de réalisation, un chitosane de degré de déacétylation d'environ 80% est utilisé.

On appelle Mw ou « masse moléculaire », la masse moléculaire moyenne en masse du polysaccharide, mesurée en Daltons.

Dans un mode de réalisation, la masse moléculaire du polysaccharide est comprise dans un intervalle de 0,01 MDa à 4,0 MDa.

Dans un mode de réalisation, la masse moléculaire du polysaccharide est comprise dans un intervalle de 0,1 MDa à 3,6 MDa.

Dans un mode de réalisation, la masse moléculaire du polysaccharide est comprise dans un intervalle de 0,10 MDa à 0,15 MDa.

Dans un mode de réalisation, la masse moléculaire du polysaccharide est comprise dans un intervalle de 0,9 MDa à 2 MDa.

Dans un mode de réalisation, la masse moléculaire du polysaccharide est comprise dans un intervalle de 2,5 à 3,6 MDa.

Dans un mode de réalisation, la masse moléculaire Mw du polysaccharide est de 2,7 MDa.

Dans un mode de réalisation, la masse moléculaire Mw du polysaccharide est de 1,5 MDa.

Dans un mode de réalisation, la masse moléculaire Mw du polysaccharide est de 1,0 MDa.

Dans un mode de réalisation, la masse moléculaire Mw du polysaccharide est de 120000 Da.

Dans un mode de réalisation, le procédé selon l'invention est caractérisé en ce que l'agent de réticulation est bi- ou polyfonctionnel.

Dans un mode de réalisation, le procédé selon l'invention est caractérisé en ce que l'agent de réticulation bi-

ou polyfonctionnel possède au moins une fonction époxyde.

Dans un mode de réalisation, le procédé selon l'invention est caractérisé en ce que l'agent de réticulation bi- ou polyfonctionnel est choisi parmi le groupe constitué par l'éther d'éthylèneglycoldiglycidyle, l'éther de butanedioldiglycidyle, l'éther de polyglycérolpolyglycidyle, l'éther de polyéthylèneglycoldiglycidyle, l'éther de polypropylèneglycoldiglycidyle, un bis- ou polyépoxy tel que 1,2,3,4-diépoxybutane ou 1,2,7,8-diépoxyoctane.

Dans un mode de réalisation, le procédé selon l'invention est caractérisé en ce que l'agent de réticulation bi- ou polyfonctionnel est l'épichlorohydrine.

Dans un mode de réalisation, le procédé selon l'invention est caractérisé en ce que l'agent de réticulation bi- ou polyfonctionnel possède au moins une fonction vinyle.

Dans un mode de réalisation, le procédé selon l'invention est caractérisé en ce que l'agent de réticulation bi- ou polyfonctionnel est une dialkylsulfone, dans laquelle les groupes alkyles, identiques ou différents, linéaires ou ramifiés sont des chaines de 1 à 4 atomes de carbone.

Dans un mode de réalisation, le procédé selon l'invention est caractérisé en ce que l'agent de réticulation bi- ou polyfonctionnel est la divinylsulfone.

Dans un mode de réalisation, le procédé selon l'invention est caractérisé en ce que l'agent de réticulation est un mono-, bi- ou polyaldéhyde.

Dans un mode de réalisation, le procédé selon l'invention est caractérisé en ce que l'agent de réticulation est le formaldéhyde.

Dans un mode de réalisation, le procédé selon l'invention est caractérisé en ce que l'agent de réticulation est le glutaraldéhyde.

Dans un mode de réalisation, le procédé selon l'invention est caractérisé en ce que le ratio molaire l'agent réticulant et le polysaccharide mis en oeuvre est compris dans un intervalle de 0,001 à 0,5.

Dans un mode de réalisation, le procédé selon l'invention est caractérisé en ce que le ratio molaire entre l'agent réticulant et le polysaccharide mis en oeuvre est compris dans un intervalle de 0,01 à 0,3.

Dans un mode de réalisation, le procédé selon l'invention est caractérisé en ce que le ratio molaire entre l'agent réticulant et le polysaccharide mis en oeuvre est compris dans un intervalle de 0,05 a 0,2.

Dans un mode de réalisation, le procédé selon l'invention est caractérisé en ce que le ratio molaire entre l'agent réticulant et le polysaccharide mis en oeuvre est égal à 0,07.

Dans un mode de réalisation, le procédé selon l'invention est caractérisé en ce que le ratio molaire entre l'agent réticulant et le polysaccharide mis en oeuvre est égal à 0,08.

Dans un mode de réalisation, le procédé selon l'invention est caractérisé en ce que le ratio molaire entre l'agent réticulant et le polysaccharide mis en oeuvre est égal à 0,10.

Dans un mode de réalisation, le procédé selon l'invention est caractérisé en ce que le ratio molaire entre l'agent réticulant et le polysaccharide mis en oeuvre est égal à 0,14.

Dans un mode de réalisation, le procédé selon l'invention est caractérisé en ce que le ratio molaire entre l'agent réticulant et le polysaccharide mis en oeuvre est égal à 0,21.

Dans un mode de réalisation, le procédé selon l'invention est caractérisé en ce que le précurseur du substituant est choisi dans le groupe des molécules comprenant une seule fonction réactive choisie dans le groupe constitué des fonctions vinyle, époxyde, allyle, cétone, aldéhyde, thiocyanate, halide, isocyanate, halosilicium, nitrile, sultone.

On entend par fonction réactive, une fonction susceptible de former une liaison avec une fonction hydroxyle du polysaccharide.

Dans un mode de réalisation, la liaison est formée par création d'une liaison éther.

Dans un mode de réalisation, la liaison est formée par création d'une liaison hémiacétal.

Dans un mode de réalisation, la liaison est formée par la création d'une liaison uréthane.

Dans les conditions du procédé selon l'invention, la formation d'une fonction ester est ainsi exclue.

Dans un mode de réalisation, le procédé selon l'invention est caractérisé en ce que le précurseur du substituant est choisi dans le groupe constitué des molécules comprenant, en outre, au moins une fonction ou un groupement d'intérêt, inertes vis-à-vis des réactions de substitution et réticulation, choisis dans le groupe constitué par les groupements ou fonctions sulfonate, alkyle linéaire ou ramifié, aromatique substitué ou non, sulfate, thiol, ose, phosphate, phosphonate, carbonate et ester.

On entend par inerte, une fonction qui ne réagit pas dans les conditions de mise en oeuvre du procédé et qui est stable dans les conditions de stockage du produit obtenu selon le procédé de l'invention. Une fonction qui, dans les conditions de mise en oeuvre du procédé, serait susceptible de ne réagir avec aucune des fonctions du polysaccharide ni avec aucune des fonctions du réticulant ni avec aucune des fonctions réactives du précurseur du substituant, est donc inerte dans les conditions de mise en oeuvre du procédé.

Dans un mode de réalisation, le procédé selon l'invention est caractérisé en ce que le précurseur du substituant est choisi dans le groupe constitué des molécules de formule générale $F\text{-}R\text{-}(G)_x$ dans laquelle :

- F est une fonction réactive choisie dans le groupe constitué des fonctions vinyle substitutée ou non, époxyde substitutée ou non, allyle substituée ou non, cétone, aldéhyde, thiocyanate, halide, isocyanate, halosilicium, nitrile, sultone ; R est une liaison ou une chaine alkyle de 1 à 12 atomes de carbone, linéaire ou ramifiée, aromatique substitué ou non, saturée ou insaturée comprenant éventuellement un ou plusieurs hétéroatomes ;
- G est soit un hydrogène, soit une fonction ou un groupement d'intérêt, inertes, choisis dans le groupe constitué par les groupements ou fonctions sulfonate, alkyle linéaire ou ramifié, aromatique substitué ou non, sulfate, thiol, ose, phosphate, phosphonate, carbonate et ester ;
- x, entier naturel tel que 1 ≤ x ≤ 3.

Dans un mode de réalisation, le procédé selon l'invention est caractérisé en ce que F est une fonction vinyle et le précurseur du substituant est choisi dans le groupe constitué par les composés de formule :

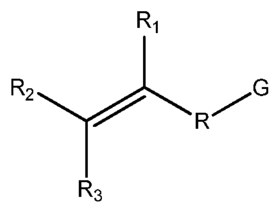

- R et G étant tels que définis ci-desssus
- $R_1$, $R_2$ et $R_3$ identiques ou différents étant soit un atome d'hydrogène, soit une chaine alkyl de 1 à 3 atomes de carbone.

Dans un mode de réalisation, le procédé selon l'invention est caractérisé en ce que F est une fonction époxyde et le précurseur du substituant est choisi dans le groupe constitué par les composés de formule :

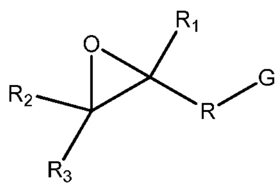

- R et G étant tels que définis ci-desssus
- $R_1$, $R_2$, $R_3$ étant tels que définis ci-dessus.

Dans un mode de réalisation, le procédé selon l'invention est caractérisé en ce que F est une fonction allyle et le précurseur du substituant est choisi dans le groupe constitué par les composés de formule :

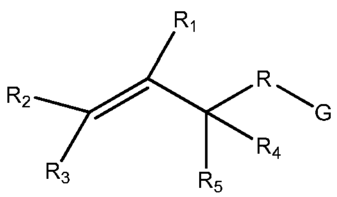

- R et G étant tels que définis ci-desssus
- $R_1$, $R_2$ et $R_3$ étant tels que définis ci-dessus
- $R_4$ et $R_5$ identiques ou différents étant soit un atome d'hydrogène, soit une chaine alkyl de 1 à 3 atomes de carbone.

Dans un mode de réalisation, le procédé selon l'invention est caractérisé en ce que G est une fonction sulfate.

Dans un mode de réalisation, le procédé selon l'invention est caractérisé en ce que G est un atome d'hydrogène.

Dans un mode de réalisation, le procédé selon l'invention est caractérisé en ce que G est une fonction sulfonate.

Dans un mode de réalisation, le procédé selon l'invention est caractérisé en ce que le substituant est choisi dans le groupe constitué par les allyle-sulfates, les époxy-sulfates, les vinyle-sulfonates, et les époxy-alcanes.

Dans un mode de réalisation, le procédé selon l'invention est caractérisé en ce que le substituant est choisi dans le groupe constitué par l'acide vinylsulfonique ou ses sels, l'époxybutane ou l'allylsulfate de sodium.

Dans un mode de réalisation, le procédé selon l'invention est caractérisé en ce que l'agent réticulant polyfonctionnel est l'éther de 1,4-butanedioldiglycidyle (BDDE) et le précurseur du substituant est le vinylsulfonate de sodium.
Dans un mode de réalisation, le procédé selon l'invention est caractérisé en ce que l'agent réticulant polyfonctionnel est l'éther de 1,4-butanedioldiglycidyle (BDDE) et le précurseur du substituant est l'allylesulfate de sodium.
Dans un mode de réalisation, le procédé selon l'invention est caractérisé en ce que l'agent réticulant polyfonctionnel est l'éther de 1,4-butanedioldiglycidyle (BDDE) et le précurseur du substituant est l'époxybutane.
Dans un mode de réalisation, le procédé selon l'invention est caractérisé en ce que l'agent réticulant polyfonctionnel est la divinylsulfone et le précurseur du substituant est le vinylsulfonate de sodium.
Dans un mode de réalisation, le procédé selon l'invention est caractérisé en ce que le ratio molaire entre le précurseur du substituant et le polysaccharide mis en oeuvre, est compris dans un intervalle de 0,001 à 4,00.
Dans un mode de réalisation, le procédé selon l'invention est caractérisé en ce que le ratio molaire entre le précurseur du substituant et le polysaccharide mis en oeuvre, est compris dans un intervalle de 0,20 à 2,20.
Dans un mode de réalisation, le procédé selon l'invention est caractérisé en ce que le ratio molaire entre le précurseur du substituant et le polysaccharide mis en oeuvre, est égal à 0,24.
Dans un mode de réalisation, le procédé selon l'invention est caractérisé en ce que le ratio molaire entre le précurseur du substituant et le polysaccharide mis en oeuvre, est égal à 0,30.
Dans un mode de réalisation, le procédé selon l'invention est caractérisé en ce que le ratio molaire entre le précurseur du substituant et le polysaccharide mis en oeuvre, est égal à 0,35.
Dans un mode de réalisation, le procédé selon l'invention est caractérisé en ce que le ratio molaire entre le précurseur du substituant et le polysaccharide mis en oeuvre, est égal à 0,90.
Dans un mode de réalisation, le procédé selon l'invention est caractérisé en ce que le ratio molaire entre le précurseur du substituant et le polysaccharide mis en oeuvre, est égal à 1,00.
Dans un mode de réalisation, le procédé selon l'invention est caractérisé en ce que le ratio molaire entre le précurseur du substituant et le polysaccharide mis en oeuvre, est égal à 1,60.
Dans un mode de réalisation, le procédé selon l'invention est caractérisé en ce que le ratio molaire entre le précurseur du substituant et le polysaccharide mis en oeuvre, est égal à 2,00.
Le procédé selon l'invention est caractérisé principalement en ce que les réactions de réticulation et de substitution sont mises en oeuvre de manière simultanée, dans les mêmes conditions expérimentales et sur les mêmes sites réactionnels du polysaccharide et ce, sans qu'il y ait compétition entre les différentes espèces mises en jeu. Les taux de substitution et de réticulation sont les mêmes que ceux des réactions réalisées de manière isolée.
Le procédé permet ainsi de pouvoir contrôler la réticulation indépendamment de la substitution pour faciliter la préparation des gels et pouvoir adapter facilement le produit en fonction de son utilisation.
La présente invention, de par la simultanéité de la substitution et de la réticulation, permet de limiter le temps de présence du polysaccharide dans un milieu alcalin qui le dégrade si le séjour est prolongé. Il est en effet bien connu que dans le cas d'une substitution simple en milieu alcalin par exemple, l'acide hyaluronique se dégrade rapidement et perd toutes ses propriétés gélifiantes et viscoélastiques. Un effet surprenant de l'invention est que la simultanéité des réactions de réticulation et de substitution protège le polysaccharide en cours de réaction et permet d'obtenir un effet synergique sur les propriétés rhéologiques, notamment l'élasticité du polysaccharide qui est fortement augmentée.
Les avantages liés à la simultanéité des réactions de réticulation/substitution ne se limitent pas à des avantages visibles sur le produit final tels qu'une meilleure élasticité, une bonne homogénéité du gel, une répartition homogène des substituants ou bien encore la limitation de la dégradation du polysaccharide lors de la réticulation/substitution, mais aussi la durée de réaction et surtout le nombre d'étapes réactionnelles. Le procédé de l'invention permet l'introduction simultanée de tous les réactifs. Une seule et unique étape réactionnelle offre non seulement un gain de temps considérable, mais limite aussi les pertes de temps et de solvants. Toutes les réactions mises en jeu, à savoir réactions de réticulation et de substitution, se produisant dans les mêmes conditions, le catalyseur introduit sera actif pour les deux réactions sans qu'il soit nécessaire d'augmenter sa quantité par rapport à une réticulation simple. L'absence de compétition entre réticulation et substitution évite de devoir ajouter un excédent de réactif pour compenser sa dégradation ou sa consommation trop rapide.
Le procédé de l'invention n'utilise pas de catalyseurs autres que des acides ou des bases simples, pas de solvants organiques, pas d'agents d'activation, et le bilan atomique de la réaction est bon, étant donné l'absence de formation de produit secondaire.
Ce dernier point est l'autre avantage de l'invention : le procédé de l'invention ne produit pas de produits secondaires devant être éliminés lors de la purification. Il s'agit simplement de rincer le produit pour éliminer l'excédent d'agent réticulant et de catalyseur. Compte tenu des applications des polysaccharides obtenus, notamment comme biomatériaux, cette absence de produits secondaires est un réel avantage compétitif.
Le procédé permettant d'obtenir les composés de la présente invention se différencie de l'art antérieur en ce que sa mise en oeuvre simple permet de façon surprenante de substituer et de réticuler un polysaccharide de façon simultanée sans que la substitution soit en compétition avec la réticulation. On contrôle ainsi mieux le taux de réticulation

ou le degré de substitution.

Le procédé permettant d'obtenir les produits de la présente invention offre une totale liberté quant au paramétrage de chacune des réactions intervenant de façon simultanée et indépendante dans le milieu réactionnel. Il est ainsi possible de modifier le taux de réticulation sans influer sur la substitution ; et inversement, il est possible de modifier le degré de substitution sans influer sur la réticulation.

La mise en oeuvre du procédé de réticulation de l'invention permet d'obtenir un produit d'une grande homogénéité, facilement injectable. De façon surprenante, le procédé selon l'invention permet d'augmenter les propriétés rhéologiques des polysaccharides réticulés sans avoir à mettre en oeuvre plus de réticulant. Il permet en outre d'apporter d'autres propriétés telles que l'hydratation, la lipophilie.

Le procédé de l'invention est tel qu'il est envisageable de faire jusqu'à trois réactions simultanément. On entend par « trois réactions simultanées » une réticulation en simultané d'une double substitution.

Dans un mode de réalisation, le procédé selon l'invention est caractérisé en ce que le polysaccharide obtenu par le procédé est substitué sur ses fonctions hydroxyles.

Le degré de substituant introduit (DSI) est défini comme étant :

$$DSI = \frac{\text{(Nombre de moles de fonctions réactives du substituant introduites dans le milieu réactionnel)}}{\text{(Nombre de moles de motif disaccharidique introduites dans le milieu réactionnel)}}$$

Dans un mode de réalisation, le procédé selon l'invention est caractérisé en ce que le degré de substituant introduit est compris dans un intervalle de 0,001 à 4,00 ($0,001 \leq DSI \leq 4,00$).

Dans un mode de réalisation, le procédé selon l'invention est caractérisé en ce que le degré de substituant introduit est compris dans un intervalle de 0,20 à 2,20 ($0,20 \leq DSI \leq 2,20$).

Dans un mode de réalisation, le procédé selon l'invention est caractérisé en ce que le degré de substituant introduit est égal à 0,24.

Dans un mode de réalisation, le procédé selon l'invention est caractérisé en ce que le degré de substituant introduit est égal à 0,30.

Dans un mode de réalisation, le procédé selon l'invention est caractérisé en ce que le degré de substituant introduit est égal à 0,35.

Dans un mode de réalisation, le procédé selon l'invention est caractérisé en ce que le degré de substituant introduit est égal à 0,90.

Dans un mode de réalisation, le procédé selon l'invention est caractérisé en ce que le degré de substituant introduit est égal à 1,00.

Dans un mode de réalisation, le procédé selon l'invention est caractérisé en ce que le degré de substituant introduit est égal à 1,60.

Dans un mode de réalisation, le procédé selon l'invention est caractérisé en ce que le degré de substituant introduit est égal à 2,00.

Dans un mode de réalisation, le procédé selon l'invention est caractérisé en ce que le polysaccharide obtenu est réticulé par réaction de l'agent réticulant sur ses fonctions hydroxyles.

Le degré de réticulant introduit (DRI) est défini comme étant :

$$DRI = \frac{\text{(Nombre de moles de réticulant introduites dans le milieu réactionnel)}}{\text{(Nombre de moles de motif dissacharidique introduites dans le milieu réactionnel)}}$$

Dans un mode de réalisation, le procédé selon l'invention est caractérisé en ce que le degré de réticulant introduit est compris dans un intervalle de 0,001 à 0,5.

Dans un mode de réalisation, le procédé selon l'invention est caractérisé en ce que le degré de réticulant introduit est compris dans un intervalle de 0,01 à 0,3.

Dans un mode de réalisation, le procédé selon l'invention est caractérisé en ce que le degré de réticulant introduit est de 0,07.

Dans un mode de réalisation, le procédé selon l'invention est caractérisé en ce que le degré de réticulant introduit est de 0,08.

Dans un mode de réalisation, le procédé selon l'invention est caractérisé en ce que le degré de réticulant introduit est de 0,10.

Dans un mode de réalisation, le procédé selon l'invention est caractérisé en ce que le degré de réticulant

introduit est de 0,14.

Dans un mode de réalisation, le procédé selon l'invention est caractérisé en ce que le degré de réticulant introduit est de 0,21.

Dans un mode de réalisation, le procédé selon l'invention est caractérisé en ce que le ratio entre la masse de polysaccharide et la masse d'eau mises en oeuvre est compris dans un intervalle de 4 à 20 %, en pourcentage massique.

Dans un mode de réalisation, le procédé selon l'invention est caractérisé en ce que le ratio entre la masse de polysaccharide et la masse d'eau mises en oeuvre est compris dans un intervalle de 6 à 16 %, en pourcentage massique.

Dans un mode de réalisation, le procédé selon l'invention est caractérisé en ce que le ratio entre la masse de polysaccharide et la masse d'eau mises en oeuvre est compris dans un intervalle de 8 à 14 %, en pourcentage massique.

Dans un mode de réalisation, le procédé selon l'invention est caractérisé en ce que le ratio entre la masse de polysaccharide et la masse d'eau mises en oeuvre est de 5,7 %, en pourcentage massique

Dans un mode de réalisation, le procédé selon l'invention est caractérisé en ce que le ratio entre la masse de polysaccharide et la masse d'eau mises en oeuvre est de 6,3 %, en pourcentage massique.

Dans un mode de réalisation, le procédé selon l'invention est caractérisé en ce que le ratio entre la masse de polysaccharide et la masse d'eau mises en oeuvre est de 10,3 %, en pourcentage massique.

Dans un mode de réalisation, le procédé selon l'invention est caractérisé en ce que le ratio entre la masse de polysaccharide et la masse d'eau mises en oeuvre est de 11,1 %, en pourcentage massique.

Dans un mode de réalisation, le procédé selon l'invention est caractérisé en ce que le ratio entre la masse de polysaccharide et la masse d'eau mises en oeuvre est de 12,2 %, en pourcentage massique.

Dans un mode de réalisation, le procédé selon l'invention est caractérisé en ce que le ratio entre la masse de polysaccharide et la masse d'eau mises en oeuvre est de 13,5 %, en pourcentage massique.

Dans un mode de réalisation, le procédé selon l'invention est caractérisé en ce que le ratio entre la masse de polysaccharide et la masse d'eau mises en oeuvre est de 14,3 %, en pourcentage massique.

Dans un mode de réalisation, le procédé selon l'invention est caractérisé en ce que le ratio entre la masse de polysaccharide et la masse d'eau mises en oeuvre est de 15,8 %, en pourcentage massique.

Dans un mode de réalisation, le procédé selon l'invention est caractérisé en ce qu'il est mis en oeuvre à température ambiante.

On entend par température ambiante une température comprise entre 18°C et 25°C.

Dans un mode de réalisation, le procédé selon l'invention est caractérisé en ce qu'il est mis en oeuvre à une température supérieure à 25°C.

Dans un mode de réalisation, le procédé selon l'invention est caractérisé en ce qu'il est mis en oeuvre à une température inférieure 60°C.

Dans un mode de réalisation, le procédé selon l'invention est caractérisé en ce qu'il est mis en oeuvre à une température comprise entre 39°C et 60°C.

Dans un mode de réalisation, le procédé selon l'invention est caractérisé en ce qu'il est mis en oeuvre à une température de 40°C.

Dans un mode de réalisation, le procédé selon l'invention est caractérisé en ce qu'il est mis en oeuvre à une température de 50°C.

Dans un mode de réalisation, le procédé selon l'invention est caractérisé en ce qu'il est mis en oeuvre pendant une durée comprise dans un intervalle de 15 minutes à 48 heures.

Dans un mode de réalisation, le procédé selon l'invention est caractérisé en ce qu'il est mis en oeuvre pendant une durée de 1 à 2 heures.

Dans un mode de réalisation, le procédé selon l'invention est caractérisé en ce qu'il est mis en oeuvre pendant une durée de 2 à 3 heures.

Dans un mode de réalisation, le procédé selon l'invention est caractérisé en ce qu'il est mis en oeuvre pendant une durée de 3 à 4 heures.

Dans un mode de réalisation, le procédé selon l'invention est caractérisé en ce qu'il est mis en oeuvre pendant une durée de 4 à 5 heures.

Dans un mode de réalisation, le procédé selon l'invention est caractérisé en ce qu'il comprend en outre une étape de lavage du polysaccharide obtenu.

Dans un mode de réalisation, le procédé selon l'invention est caractérisé en ce qu'il comprend en outre une étape de lavage du polysaccharide obtenu avec une solution tampon ayant un pH d'environ 7.

Dans un mode de réalisation, le procédé selon l'invention est caractérisé en ce qu'il comprend en outre une étape de lavage du polysaccharide obtenu avec de l'eau purifiée.

Dans un mode de réalisation, le produit obtenu selon le procédé de l'invention est caractérisé en ce que la réticulation du polysaccharide est réalisée par des ponts diéthylsulfones de formule $PS\text{-}O\text{-}(CH_2)_n\text{-}S(O_2)\text{-}(CH_2)_n\text{-}O\text{-}PS$, où « PS » représente le reste polysaccharide, n entier tel que $1 \leq n \leq 4$.

Dans un mode de réalisation, le produit obtenu selon le procédé de l'invention est caractérisé en ce que la

réticulation du polysaccharide est réalisée par des ponts diéthylsulfones de formule PS-O-$CH_2$-$CH_2$-S($O_2$)-$CH_2$-$CH_2$-O-PS.

Dans un mode de réalisation, le produit obtenu selon le procédé de l'invention est caractérisé en ce que la réticulation du polysaccharide est réalisée par des ponts de formule PS-O-$CH_2$-CH(OH)-$CH_2$-X-$CH_2$-CH(OH)-$CH_2$-O-PS ; le groupe X étant soit une chaine alkyles de 2 à 6 atomes de carbone, soit une chaine polyéther.

Dans un mode de réalisation, le produit obtenu selon le procédé de l'invention est caractérisé en ce que la réticulation du polysaccharide est réalisée par des ponts éthers de formule PS-O-$CH_2$-O-PS.

Dans un mode de réalisation, le produit obtenu selon le procédé de l'invention est caractérisé en ce que la réticulation du polysaccharide est réalisée par des ponts hémiacétals de formule PS-O-CH(OH)-$(CH_2)_m$-CH(OH)-O-PS, m entier tel que $0 \leq m \leq 4$.

Dans un mode de réalisation, le produit obtenu selon le procédé de l'invention est caractérisé en ce que le polysaccharide porte sur au moins une de ses fonctions hydroxyles, au moins un substituant issu de l'acide vinylsulfonique, l'acide 2-éthoxyéthylsulfonique.

Dans un mode de réalisation, le produit obtenu selon le procédé de l'invention est caractérisé en ce que le polysaccharide porte sur au moins une de ses fonctions hydroxyles, au moins un substituant issu de l'époxybutane, le 1-éthoxybutan-2-ol.

Dans un mode de réalisation, le produit obtenu selon le procédé de l'invention est caractérisé en ce que le polysaccharide porte sur au moins une de ses fonctions hydroxyles, au moins un substituant issu de l'allylsulfate de sodium, le 3-propoxysulfate de sodium.

L'invention concerne l'utilisation d'un hydrogel obtenu selon le procédé de l'invention, pour la formulation d'une composition de viscosupplémentation.

Le procédé selon l'invention concerne également les compositions comprenant un polysaccharide obtenu par le procédé selon l'invention.

Dans un mode de réalisation, le polysaccharide obtenu par le procédé selon l'invention est sous forme de gel ou d'hydrogel.

À l'issue de la réticulation et de la substitution, il peut être avantageux de neutraliser le gel obtenu, selon les procédés standards connus dans le domaine, et par exemple par ajout d'acide lorsque le procédé est conduit en milieu basique, et par ajout d'une base, lorsque le procédé est conduit en milieu acide.

Le mélange obtenu à l'issue du procédé de l'invention peut être éventuellement soumis à une étape d'hydratation complémentaire, afin d'obtenir un gel sous forme d'hydrogel injectable, adapté aux applications envisagées.

Cette hydratation est généralement effectuée, dans un milieu aqueux, par simple mélange du gel réticulé et substitué avec une solution aqueuse, avantageusement physiologique tamponnée, de manière à obtenir une concentration finale, variable dans de très large proportions, selon la nature des polysaccharides utilisés, leurs taux de réticulation respectifs, et également selon l'utilisation envisagée. La solution tamponnée utilisable peut par exemple être une solution physiologique, iso-osmolaire présentant un pH compris entre environ 6,8 et environ 7,5.

Cette concentration finale en polysaccharides totaux est généralement comprise entre environ 5 et environ 100 mg/g, de préférence entre environ 5 et environ 50 mg/g, par exemple environ 20 mg/g d'hydrogel.

L'invention concerne l'utilisation d'un polysaccharide obtenu selon le procédé de l'invention, pour la formulation d'une composition de viscosupplémentation.

L'invention concerne l'utilisation d'un polysaccharide obtenu selon le procédé de l'invention, pour la formulation d'une composition pour le comblement des rides.

Les applications visées sont plus particulièrement les applications communément observées dans le cadre des viscoélastiques injectables de polysaccharides utilisés ou potentiellement utilisables dans les pathologies ou traitements suivants :

- injections esthétiques : comblement des rides, défauts cutanés ou volumatrices (pommettes, mentons, lèvres) ;
- traitement de l'arthrose, injection dans l'articulation en remplacement ou complément du liquide synovial déficient ;
- injection péri-urétrale pour le traitement de l'incontinence urinaire par insuffisance sphinctérienne ;
- injection post-chirurgicale pour éviter les adhésions péritonéales notamment ;
- injection suite à une chirurgie de la presbytie par incisions sclérales au laser ;
- injection dans la cavité vitréenne.

Plus particulièrement, en chirurgie esthétique, en fonction de ses propriétés viscoélastiques et de rémanence, l'hydrogel obtenu selon le procédé de l'invention pourra être utilisé :

- pour le comblement des rides fines, moyennes ou profondes, et être injecté avec des aiguilles de diamètre fin (27 Gauge par exemple) ;
- comme volumateur avec une injection par des aiguilles de diamètre plus important, de 22 à 26 Gauge par exemple,

et plus longues (30 à 40 mm par exemple); dans ce cas, son caractère cohésif permettra de garantir son maintien à l'emplacement de l'injection.

Le polysaccharide obtenu selon le procédé de l'invention trouve également une application importante en chirurgie articulaire et en chirurgie dentaire pour le comblement des poches parodontales par exemple.

Ces exemples d'utilisation ne sont nullement limitants, le polysaccharide obtenu selon le procédé de la présente invention étant plus largement prévu pour :

- combler des volumes ;
- générer des espaces au sein de certains tissus, favorisant ainsi leur fonctionnement optimal ;
- remplacer des liquides physiologiques déficients.

Le polysaccharide obtenu selon le procédé de l'invention trouve également une application pour la préparation de substituts osseux.

Le polysaccharide obtenu selon le procédé de l'invention peut également trouver une application tout à fait intéressante en tant que matrice de relargage d'un (ou plusieurs) principe(s) actif(s) au préalable dispersé(s) en son sein. Par principe actif, on entend tout produit actif sur le plan pharmacologique : principe actif médicamenteux, antioxydant (sorbitol, mannitol ...), antiseptique, anti-inflammatoire, anesthésiques locaux (lidocaine...), etc.

De manière pratique, le polysaccharide obtenu selon le procédé de l'invention, de préférence après purification et hydratation en hydrogel, peut être conditionné, par exemple dans des seringues, et stérilisé selon tout moyen connu en soi (par exemple par autoclavage) pour commercialisation et/ou utilisation directe.

Selon un autre aspect, la présente invention concerne un kit comprenant un polysaccharide obtenu selon le procédé de l'invention, conditionné en seringue stérile.

Les caractéristiques des polysaccharides obtenus selon le procédé de l'invention sont mises en évidence dans les exemples ci-après.

**Exemples**

Les degrés de substituant introduit (DSI), les degrés de réticulant introduit (DRI) et les ratios catalyseurs réactionnels dans les exemples qui suivent sont définis par :

Degré de réticulant introduit :

DRI = Nombre de moles de réticulant introduites dans le milieu réactionnel / nombre de moles de motif disaccharidique introduites dans le milieu réactionnel

Degré de substituant introduit:

DSI = Nombre de moles de fonctions réactives du substituant introduites dans le milieu réactionnel / nombre de moles de motif disaccharidique introduites dans le milieu réactionnel

Ratio Catalyseur Réactionnel

RCR = Nombre de moles de fonctions réactives du catalyseur introduites dans le milieu réactionnel / nombre de moles de motif disaccharidique introduites dans le milieu réactionnel

**Exemple 1** :

**Mise en évidence de l'effet synergique sur les propriétés rhéologiques de la substitution effectuée simultanément à la réticulation.**

Les étapes suivantes sont ci-après décrites :

- ○ Substitution du VSA (sel de sodium d'acide vinylsulfonique) sur NaHA (hyaluronate de sodium) non réticulé
- ○ Caractérisation de la substitution
- ○ Réticulation du NaHA par du BDDE (éther de 1,4-butanedioldiglycidyle)
- ○ Substitution du VSA (sel de sodium d'acide vinylsulfonique) sur NaHA simultanément à une réticulation par du BDDE (éther de 1,4-butanedioldiglycidyle)
- ○ Substitution du VSA (sel de sodium d'acide vinylsulfonique) sur NaHA suivi d'une réticulation par du BDDE (éther de 1,4-butanedioldiglycidyle)
- ○ Mise en évidence de l'effet synergique des propriétés rhéologiques apportées par la substitution mise en oeuvre simultanément à la réticulation

**• Synthèse Gel G1A : Substitution du VSA (sel de sodium d'acide vinylsulfonique) sur NaHA non réticulé, à une température de 50°C et en milieu alcalin (RCR = 0,8:1)**

Etape a) : Hydratation des fibres de hyaluronate de sodium sous forme de gel non réticulé

Des fibres de hyaluronate de sodium de qualité injectable ( 0,9 g soit 2,24 mmol ; masse moléculaire : environ 2,7 MDa) ; sont pesées dans un récipient. Une solution aqueuse d'hydroxyde de sodium à 1 % (0,25 mol/L soit 1,85 mmol de $HO^-$ introduites dans le milieu) dans l'eau (7,4 g) est ajoutée (RCR = 0,8:1), le tout est homogénéisé pendant environ 1 heure à la spatule, à température ambiante et 900 mmHg.

Etape b) : Substitution

Du VSA (102 mg soit 0.78 mmol) est ajouté au gel de hyaluronate de sodium (NaHA) non réticulé obtenu à l'étape précédente, le tout étant homogénéisé à la spatule pendant environ 30 minutes à température 12-14°C. L'ensemble est ensuite placé au bain-marie à 50°C pendant 2h10. Le degré de substituant introduit DSI est égal à environ 0,35.

Etape c) : Neutralisation, purification

Le gel final substitué est ensuite neutralisé par l'ajout d'HCl 1N, et placé dans un bain de tampon phosphate pour stabiliser le pH et permettre son hydratation, ou gonflement pour obtenir un gel comprenant 20 mg/g de HA (acide hyaluronique), ce gel est ensuite homogénéisé avant d'être conditionné en seringues qui sont stérilisées par autoclavage. On obtient ainsi un hydrogel G1A de NaHA substitué et stérilisé.

**• Synthèse Gel REF1A**

Le gel REF1A est synthétisé suivant le mode opératoire du gel G1A décrit ci-dessus, en remplaçant le VSA par de l'eau pour injection parentérale (EPPI).

**• Caractérisation des modifications chimiques des gels G1A et REF1A par RMN $^1$H liquide:**

Le spectre RMN du Gel G1A est représenté à la figure 1.

Le spectre RMN du Gel REF1A est représenté à la figure 2.

La comparaison des spectres de figures 1 et 2 permet de confirmer la substitution chimique (par liaison covalente) du VSA sur la fonction hydroxyle du NaHA.

• **Synthèse Gel G1B** : **Réticulation du NaHA par du BDDE (éther de 1,4-butanedioldiglycidyle), à une température de 50°C et en milieu alcalin (RCR = 0,8:1)**

Etape a) : Identique à l'étape a) de synthèse du gel G1A

Etape b) : Réticulation

Du BDDE (65 mg soit 0,32 mmol) est ajouté au gel de hyaluronate de sodium (NaHA) non réticulé obtenu à l'étape précédente, le tout étant homogénéisé à la spatule pendant environ 30 minutes à température 12-14°C. L'ensemble est ensuite placé au bain-marie à 50°C pendant 2h10. Le degré de réticulant introduit DRI est égal à environ 0,14.

Etape c) : Neutralisation, purification

Le gel final réticulé est ensuite neutralisé par l'ajout d'HCl 1N, et placé dans un bain de tampon phosphate pour stabiliser le pH et permettre son hydratation, ou gonflement pour obtenir un gel comprenant 20 mg/g de HA, ce gel est ensuite homogénéisé avant d'être rempli en seringues qui sont stérilisées par autoclavage. On obtient ainsi un hydrogel G1B de NaHA réticulé et stérilisé.

• **Synthèse Gel G1C : Substitution du VSA (sel de sodium d'acide vinylsulfonique) sur NaHA en simultanément à une réticulation par du BDDE (éther de 1,4-butanedioldiglycidyle), à une température de 50°C et en milieu alcalin (RCR = 0,8:1)**

Etape a) : Identique à l'étape a) de synthèse du gel G1A

Etape b) : Réticulation et Substitution

Du BDDE (65 mg soit 0,32 mmol) et du VSA (102 mg soit 0,78 mmol) sont ajoutés au gel de hyaluronate de sodium (NaHA) non réticulé obtenu à l'étape précédente, le tout étant homogénéisé à la spatule pendant environ 30 minutes à température 12-14°C. L'ensemble est ensuite placé au bain-marie à 50°C pendant 2h10. Le degré de réticulant introduit DRI est égal à environ 0,14 et le degré de substituant introduit DSI est égal à environ 0,35.

Etape c) : Neutralisation, purification

Le gel final réticulé et substitué est ensuite neutralisé par l'ajout d'HCl 1N, et placé dans un bain de tampon phosphate pour stabiliser le pH et permettre son hydratation, ou gonflement pour obtenir un gel comprenant 20 mg/g de HA, ce gel est ensuite homogénéisé avant d'être conditionné en seringues qui sont stérilisées par autoclavage. On obtient ainsi un hydrogel G1C de NaHA réticulé, substitué et stérilisé.

• **Synthèse Gel G1D : Substitution du VSA (sel de sodium d'acide vinylsulfonique) sur NaHA suivi d'une réticulation par du BDDE (éther de 1,4-butanedioldiglycidyle), à une température de 40°C et en milieu alcalin (RCR = 0,8:1)**

Etape a) : Identique à l'étape a) de synthèse du gel G1A

Etape b) : Substitution

Du VSA (102 mg soit 0.78 mmol) est ajouté au gel de hyaluronate de sodium (NaHA) non réticulé obtenu à l'étape précédente, le tout étant homogénéisé à la spatule pendant environ 30 minutes à température 12-14°C. L'ensemble est ensuite placé au bain-marie à 40°C pendant 1h00. Le degré de substituant introduit DSI est égal à environ 0,35. Le temps et la température ont été réduits par rapport à l'essai G1A, afin de conserver un gel assez consistant pour l'étape suivante de réticulation.

Etape c) : Réticulation

Du BDDE (65 mg soit 0,32 mmol) est ajouté au gel de hyaluronate de sodium (NaHA) substitué, non réticulé, obtenu à l'étape précédente, le tout étant homogénéisé à la spatule pendant environ 30 minutes à température 12-14°C. L'ensemble est ensuite placé au bain-marie à 50°C pendant 2h10. Le degré de réticulant introduit DRI est égal à environ 0,14.

Etape d) : Neutralisation, purification

Le gel final réticulé et substitué est ensuite neutralisé par l'ajout d'HCl 1N, et placé dans un bain de tampon phosphate pour stabiliser le pH et permettre son hydratation, ou gonflement pour obtenir un gel comprenant 20 mg/g de HA, ce gel est ensuite homogénéisé avant d'être conditionné en seringues qui sont stérilisées par autoclavage. On obtient ainsi un hydrogel G1D de NaHA substitué puis réticulé et stérilisé.

**• Mise en évidence de l'effet synergique des propriétés rhéologiques apportées par la substitution simultanément à la réticulation**

La viscosité $\eta$ des gels stériles est caractérisée sur rhéomètre TA Instruments AR 2000 Ex, en contrainte imposée à 25°C. La valeur de viscosité est relevée à une contrainte de 0,02 $s^{-1}$.

La composante élastique G' et la composante visqueuse G" des gels stériles sont caractérisées sur rhéomètre TA Instruments AR 2000 Ex, en oscillation à 25°C, les valeurs des composantes élastiques et visqueuses étant relevées à une fréquence de 1 Hz.

Les résultats rhéologiques sont présentés dans le tableau I ci-dessous :

Tableau I

| | G1A | G1B | G1C | G1D |
|---|---|---|---|---|
| Viscosité : $\eta$ (Pa.s) à 0,02 $s^{-1}$ | 7 | 1822 | 1959 | 1502 |
| Composante élasticité : G' (Pa) à 1 Hz | 0,5 | 107 | 131 | 78 |
| Composante visqueuse : G" (Pa) à 1 Hz | 3 | 27 | 29 | 22 |

La substitution sur un gel simultanément à la réticulation (essai G1C) apporte des propriétés viscoélastiques supérieures par rapport aux gels:

- ○ simplement substitués (gel G1A),
- ○ simplement réticulés (gel G1B),
- ○ d'abord substitués puis réticulés (gel G1D).

De façon surprenante, la substitution mise en oeuvre simultanément à une réticulation améliore de façon synergique les propriétés rhéologiques des gels obtenus.

**Exemple 2** :

**Substitution VSA (sel de sodium d'acide vinylsulfonique) sur NaHA simultanément à une réticulation par du BDDE (éther de 1,4-butanedioldiglycidyle), à une température de 50°C et en milieu alcalin (RCR = 0,8:1)**

Cet exemple permet la mise en évidence par la mesure des propriétés rhéologiques :

- ○ de la substitution de la fonction vinyle sur le NaHA en cours de réticulation et de la différence de structure apportée par la substitution,
- ○ de la meilleure résistance à la dégradation radicalaire du gel substitué et réticulé.

**• Synthèse Gel G2**

Etape a) : Hydratation des fibres de hyaluronate de sodium sous forme de gel non réticulé

Des fibres de hyaluronate de sodium de qualité injectable ( 0,9 g soit 2,24 mmol ; masse moléculaire : environ 2,7 MDa) sont pesées dans un récipient. Une solution aqueuse d'hydroxyde de sodium à 1 % (0,25 mol/L soit 1,85 mmol de $HO^-$ introduites dans le milieu) dans l'eau (7,4 g) est ajoutée (RCR = 0,8:1), le tout est homogénéisé pendant environ 1 heure à la spatule, à température ambiante et 900 mmHg.

Etape b) : Réticulation et Substitution

Du BDDE (65 mg soit 0.32 mmol) et du VSA (70 mg soit 0,54 mmol) sont ajoutés au gel de hyaluronate de sodium (NaHA) non réticulé obtenu à l'étape précédente, le tout étant homogénéisé à la spatule pendant environ 30 minutes à température 12-14°C. L'ensemble est ensuite placé au bain-marie à 50°C pendant 2h10. Le degré de réticulant introduit DRI est égal à environ 0,14 et le degré de substituant introduit DSI est égal à environ 0,24.

Etape c) : Neutralisation, purification

Le gel final réticulé et substitué est ensuite neutralisé par l'ajout d'HCl 1N, et placé dans un bain de tampon phosphate pour stabiliser le pH et permettre son hydratation, ou gonflement pour obtenir un gel comprenant 20 mg/g de HA, ce gel est ensuite homogénéisé avant d'être conditionné en seringues qui sont stérilisées par autoclavage. On obtient ainsi un hydrogel G2 de NaHA réticulé, substitué et stérilisé.

• **Synthèse Gel REF2**

Le gel REF2 est synthétisé suivant le mode opératoire du gel G2 décrit ci-dessus, en remplaçant le VSA par de l'eau pour injection parentérale (EPPI).

• **Caractérisation de la force d'extrusion ou « injectabilité » et de l'élasticité des gels G2 et REF2**

La force d'extrusion est caractérisée sur banc de traction Mecmesin sous vitesse de compression 50 mm/min avec des aiguilles 27G ½", les résultats sont donnés dans le tableau ci-dessous.

L'élasticité des gels stériles est caractérisée sur rhéomètre TA Instruments AR 2000 Ex, en oscillation à 25°C, la valeur de l'élasticité étant relevée à une fréquence de 1 Hz, les résultats sont présentés dans le tableau II ci-dessous.

Tableau II

| | G2 | REF2 |
|---|---|---|
| Force d'extrusion (N)Aig 27G ½" Vitesse 50 mm/min | 35 | 38 |
| Elasticité : G' (Pa) à 1 Hz | 116 | 105 |

La substitution permet d'obtenir des produits finis de rhéologie plus importante (+10 %) pour des niveaux d'injectabilité légèrement plus faibles (-8 %). Ces données rhéologiques confirment la modification chimique du NaHA, donc la substitution de la fonction vinyle sur le NaHA en cours de réticulation.

• **Test de dégradation radicalaire des gels G2 et REF2**

Les gels ont également été caractérisés par un test de dégradation radicalaire in vitro à 37°C. Ce test permet de simuler la rémanence ultérieure *in vivo* (intra-dermique, intra-articulaire, ...) des gels injectés.

Il a été mis au point sur la base du test décrit dans la publication « Antioxidant activities of sulfated polysaccharides from brown and red seaweeds », Rocha de Souza, J. Appl. Phycol. (2007) 19 :153-160.

Les gels sont dégradés par les radicaux libres générés par la réaction de Fenton entre l'eau oxygénée et les ions ferreux. La dégradation est suivie par rhéologie à 37°C, en mesurant la viscosité complexe. Les courbes de tendance des résultats de dégradation de ces 2 gels permettant ensuite d'évaluer le temps de demi-vie de ces différents gels (durée nécessaire pour avoir $n^* = n^*_0 / 2$, en minutes), avec $n^*_0$ = viscosité complexe à $t_0$ du gel caractérisé. Les temps de demi-vie obtenus sont donnés dans le tableau III ci-dessous.

Tableau III

| | G2 | REF2 |
|---|---|---|
| Temps de demi-vie (minutes) | 8,0 | 5,1 |

Ainsi, pour une injectabilité légèrement plus faible et permettant de conserver une bonne maitrise du geste chirurgical, les temps de demi-vie des gels modifiés obtenus selon l'invention sont plus longs, garantissant un temps de rémanence *in vivo* supérieur, et ce même avec le degré de substitution faible testé.

**Exemple 3** :

**Substitution VSA (sel de sodium d'acide vinylsulfonique) sur NaHA simultanément à une réticulation par du BDDE (éther de 1,4-butanedioldiglycidyle), à une température de 50°C et en milieu alcalin (RCR = 0,8:1)**

Cet exemple permet la mise en évidence par la rhéologie de l'augmentation d'élasticité apportée au gel en fonction du degré de substitution.

• **Synthèse Gels G3A et G3B**

La synthèse des gels est identique à celle des gels G1C et G2, avec les quantités de VSA adaptées aux degrés de substituant introduits testés, voir tableau IV ci-après.

Tableau IV

| | DSI |
|---|---|
| G3A | 1,00 |
| G3B | 2,00 |

• **Caractérisation de l'élasticité des gels G3A et G3B**

L'élasticité des gels est caractérisée sur rhéomètre TA Instruments AR 2000 Ex, décrit en exemple 2, les résultats sont donnés dans le tableau V ci-dessous.

Tableau V

| | G2 | G1C | G3A | G3B | REF2 |
|---|---|---|---|---|---|
| Degré de substituant introduit DSI | 0,24 | 0,35 | 1,00 | 2,00 | - |
| Elasticité : G' (Pa) à 1 Hz | 116 | 131 | 199 | 213 | 105 |

Les résultats représentés de façon graphique à la figure 3, montrent que l'élasticité G' des gels augmente avec le degré de substituant introduit : la modification chimique est effectivement responsable de l'optimisation des propriétés viscoélastiques des gels.

**Exemple 4** :

**Substitution EB (époxybutane) sur NaHA en simultanément à une réticulation par du BDDE (éther de 1,4-butanedioldiglycidyle), à une température de 50°C et en milieu alcalin (RCR = 0,8:1)**

Cet exemple permet la mise en évidence par la rhéologie de la substitution d'une fonction époxy sur le NaHA simultanément à une réticulation.

• **Synthèse Gel G4**

Etape a) : Identique à l'étape a) de synthèse du gel G1A.

Etape b) : Réticulation et substitution

Du BDDE (65 mg soit 0,32 mmol) et du EB (147 mg soit 2.02 mmol) sont ajoutés au gel de hyaluronate de sodium (NaHA) non réticulé obtenu à l'étape précédente, le tout étant homogénéisé à la spatule pendant environ 30 minutes à température 12-14°C. L'ensemble est ensuite placé au bain-marie à 50°C pendant 2h10. Le degré de réticulant introduit DRI est égal à environ 0,14 et le degré de substituant introduit DSI est égal à environ 0,90.

Etape c) : Neutralisation, purification

Le gel final réticulé et substitué est ensuite neutralisé par l'ajout d'HCl 1N, et placé dans un bain de tampon

phosphate pour stabiliser le pH et permettre son hydratation, ou gonflement, pour obtenir un gel comprenant 20 mg/g de HA, ce gel est ensuite homogénéisé avant d'être conditionné en seringues. On obtient ainsi un hydrogel G4 de NaHA réticulé, substitué et stérilisé.

• **Synthèse Gel REF4**

Le gel REF4 est synthétisé suivant le mode opératoire du gel G4 décrit ci-dessus, en remplaçant le EB par de l'eau pour injection parentérale (EPPI).

• **Caractérisation de l'élasticité des gels G4 et REF4**

L'élasticité des gels est caractérisée sur rhéomètre TA Instruments AR 2000 Ex, décrit en exemple 2, les résultats sont donnés dans le tableau VI ci-dessous.

Tableau VI

| | G4 | REF4 |
|---|---|---|
| Elasticité : G' (Pa) à 1 Hz | 180 | 150 |

La substitution d'une molécule à groupement réactif époxy sur le NaHA est confirmée par les résultats rhéologiques : la modification chimique permet d'obtenir des produits finis dont l'élasticité est plus importante.

Ces données rhéologiques confirment la substitution de la fonction époxyde sur le NaHA en cours de réticulation.

**Exemple 5** :

**Substitution VSA (sel de sodium d'acide vinylsulfonique) sur NaHA simultanément à une réticulation par du BDDE (éther de 1,4-butanedioldiglycidyle), à une température de 50°C et en milieu alcalin (RCR = 0,7:1)**

Cet exemple permet la mise en évidence par la rhéologie de la substitution sur NaHA de faible poids moléculaire

• **Synthèse Gel G5**

Etape a) : Hydratation des fibres de hyaluronate de sodium sous forme de gel non réticulé

Des fibres de hyaluronate de sodium de qualité injectable ( 0,9 g soit 2,24 mmol ; masse moléculaire : environ 1,5 MDa) ; sont pesées dans un récipient. Une solution aqueuse d'hydroxyde de sodium à 1 % (0,25 mol/L soit 1,57 mmol de $HO^-$ introduites dans le milieu) dans l'eau (6,3 g) est ajoutée (RCR = 0,7:1), le tout est homogénéisé pendant environ 1 heure à la spatule, à température ambiante et 900 mmHg.

Etape b) : Réticulation et Substitution

Du BDDE (30 mg soit 0,15 mmol) et du VSA (87 mg soit 0,67 mmol) sont ajoutés au gel de hyaluronate de sodium (NaHA) non réticulé obtenu à l'étape précédente, le tout étant homogénéisé à la spatule pendant environ 30 minutes à température 12-14°C. L'ensemble est ensuite placé au bain-marie à 50°C pendant 2h10. Le degré de réticulant introduit DRI est égal à environ 0,07 et le degré de substituant introduit DSI est égal à environ 0,30.

Etape c) : Neutralisation, purification

Le gel final réticulé et substitué est ensuite neutralisé par l'ajout d'HCl 1N, et placé dans un bain de tampon phosphate pour stabiliser le pH et permettre son hydratation, ou gonflement pour obtenir un gel comprenant 20 mg/g de HA, ce gel est ensuite homogénéisé avant d'être conditionné en seringues. On obtient ainsi un hydrogel G5 de NaHA réticulé et substitué.

• **Synthèse Gel REF5**

Le gel REF5 est synthétisé suivant le mode opératoire du gel G5 décrit ci-dessus, en remplaçant le VSA par de l'eau pour injection parentérale (EPPI).

• **Caractérisation de l'élasticité des gels G5 et REF5**

L'élasticité des gels est caractérisée sur rhéomètre TA Instruments AR 2000 Ex, décrit en exemple 2, les résultats sont donnés dans le tableau VII ci-dessous.

Tableau VII

| | G5 | REF5 |
|---|---|---|
| Elasticité : G' (Pa) à 1 Hz | 519 | 433 |

La substitution permet d'obtenir des produits finis dont l'élasticité est plus importante. Ces données rhéologiques, comme celles de l'exemple 2 (NaHA de poids moléculaire élevé), confirment que la substitution peut être réalisée sur du NaHA de différents poids moléculaires.

**Exemple 6 (exemple non représentatif de l'invention)** :

**Substitution VSA (sel de sodium d'acide vinylsulfonique) sur CMC (carboxyméthylcellulose) simultanément à une réticulation par du BDDE (éther de 1,4-butanedioldiglycidyle), à une température de 50°C et en milieu alcalin (RCR = 1:1)**

Cet exemple permet la mise en évidence par la rhéologie de la substitution sur un polysaccharide autre que le NaHA

• **Synthèse Gel G6**

Etape a) : Hydratation de la CMC sous forme de gel non réticulé

0,93 g soit 2,20 mmol de CMC de sodium (fournie par Sigma, masse moléculaire : environ 1,0 MDa) est pesée dans un récipient. Une solution aqueuse d'hydroxyde de sodium à 1 % (0,25 mol/L soit 2,25 mmol de $HO^-$ introduites dans le milieu réactionnel) dans l'eau (9,0 g) est ajoutée (RCR = 1:1), le tout est homogénéisé pendant environ 90 minutes à la spatule, à température ambiante et 900 mmHg.

Etape b) : Réticulation et Substitution

Du BDDE (37 mg soit 0,18 mmol) et du VSA (87 mg soit 0,67 mmol) sont ajoutés au gel de CMC non réticulé obtenu à l'étape précédente, le tout étant homogénéisé à la spatule pendant environ 30 minutes à température 12-14°C. L'ensemble est ensuite placé au bain-marie à 50°C pendant 3h35. Le degré de réticulant introduit DRI est égal à environ 0,08 et le degré de substituant introduit DSI est égal à environ 0,30.

Etape c) : Neutralisation, purification

Le gel final réticulé et substitué est ensuite neutralisé par l'ajout d'HCl 1N, et placé dans un bain de tampon phosphate pour stabiliser le pH et permettre son hydratation, ou gonflement pour obtenir un gel comprenant 30 mg/g de CMC, ce gel est ensuite homogénéisé avant d'être conditionné en seringues. On obtient ainsi un hydrogel G6 de CMC réticulé et substitué.

• **Synthèse Gel REF6**

Le gel REF6 est synthétisé suivant le mode opératoire du gel G6 décrit ci-dessus, en remplaçant le VSA par de l'eau pour injection parentérale (EPPI).

• **Caractérisation de l'élasticité des gels G6 et REF6**

L'élasticité des gels est caractérisée sur rhéomètre TA Instruments AR 2000 Ex, décrit en exemple 2, les résultats sont donnés dans le tableau VIII ci-dessous.

Tableau VIII

| | G6 | REF6 |
|---|---|---|
| Elasticité : G' (Pa) à 1 Hz | 524 | 483 |

La substitution permet d'obtenir des produits finis dont l'élasticité est plus importante. Ces données rhéologiques, comme celles de l'exemple 2 (NaHA), confirment que la substitution peut être réalisée sur différents squelettes polysaccharidiques, notamment les dérivés cellulosiques.

**Exemple 7** :

**Substitution EB (époxybutane) sur CH (chitosane) simultanément à une réticulation par du BDDE (éther de 1,4-butanedioldiglycidyle), à une température de 50°C et en milieu acide faible (RCR = 0.06:1) et acide fort (RCR = 1:1)**

Cet exemple permet la mise en évidence par la rhéologie de la substitution en milieu acide, simultanément à la réticulation

• **Synthèse Gel G7a (en milieu acide faible)**

Etape a) : Hydratation du CH sous forme de gel non réticulé

0,99 g soit 2,93 mmol de CH de degré de déactétylation de l'ordre de 80% (fournie par Kitozyme, masse moléculaire : environ 120000 Da) est pesée dans un récipient. Une solution aqueuse **d'acide glutamique 1 %** (0,07 mol/L) dans l'eau (9,0 g) est ajoutée. L'acide glutamique étant un acide faible, il est partiellement dissocié dans l'eau. Le pH de la solution aqueuse peut être calculé via la formule suivante (déterminée suite à des approximations) : pH = (½ pKa) - (½ log [Acide Glutamique]), soit pH = (½ × 2,19) - (½ × log(0,07)) = 1,67. La concentration d'ion hydronium $H_3O^+$ peut être calculée par la formule suivante : $[H_3O^+] = 10^{-pH}$ soit $[H_3O^+]$ = 0,02 mol/L, soit 0,19 mmol d'ions hydronium introduits dans le milieu réactionnel (RCR = 0,06:1). Le tout est homogénéisé pendant environ 90 minutes à la spatule, à température ambiante et 900 mmHg. Le pH du milieu réactionnel est de 5,3.

Etape b) : Réticulation et Substitution

Du BDDE (60 mg soit 0.30 mmol) et du EB (337 mg soit 4,68 mmol) sont ajoutés au gel de CH non réticulé obtenu à l'étape précédente, le tout étant homogénéisé à la spatule pendant environ 30 minutes à température 12-14°C. L'ensemble est ensuite placé au bain-marie à 50°C pendant . Le degré de réticulant introduit DRI est égal à environ 0,10 et le degré de substituant introduit DSI est égal à environ 1,60.

Etape c) : Neutralisation, purification

Le gel final réticulé et substitué est ensuite neutralisé par l'ajout de soude 1N, et placé dans un bain de tampon phosphate pour stabiliser le pH et permettre son hydratation, ou gonflement pour obtenir un gel comprenant 22 mg/g de CH, ce gel est ensuite homogénéisé avant d'être conditionné en seringues qui sont stérilisées par autoclavage. On obtient ainsi un hydrogel G7a de CH réticulé, substitué et stérilisé.

• **Synthèse Gel G7b (en milieu acide fort)**

Etape a) : Hydratation du CH sous forme de gel non réticulé

0,99 g soit 2,93 mmol de CH de degré de déactétylation de l'ordre de 80% (fournie par Kitozyme, masse moléculaire : environ 120000 Da) est pesée dans un récipient. Une solution aqueuse d'acide chlorhydrique 1,15 % (0,32 mol/L soit 2,88 mmol d'ion $H_3O^+$ introduits dans le milieu réactionnel = > RCR = 1:1) dans l'eau (9,0 g) est ajoutée. Le tout est homogénéisé pendant environ 90 minutes à la spatule, à température ambiante et 900 mmHg. Le pH du milieu réactionnel est de 3.

Etape b) : Réticulation et Substitution

Du BDDE (60 mg soit 0,30 mmol) et du EB (337 mg soit 4,68 mmol) sont ajoutés au gel de CH non réticulé

obtenu à l'étape précédente, le tout étant homogénéisé à la spatule pendant environ 30 minutes à température 12-14°C. L'ensemble est ensuite placé au bain-marie à 50°C pendant . Le degré de réticulant introduit DRI est égal à environ 0,10 et le degré de substituant introduit DSI est égal à environ 1,60.

Etape c) : Neutralisation, purification

Le gel final réticulé et substitué est ensuite neutralisé par l'ajout de soude 1N, et placé dans un bain de tampon phosphate pour stabiliser le pH et permettre son hydratation, ou gonflement pour obtenir un gel comprenant 22 mg/g de CH, ce gel est ensuite homogénéisé avant d'être conditionné en seringues qui sont stérilisées par autoclavage. On obtient ainsi un hydrogel G7b de CH réticulé, substitué et stérilisé.

• **Synthèse Gel REF7**

Le gel REF7 est synthétisé suivant le mode opératoire du gel G7 décrit ci-dessus, en remplaçant le EB par de l'eau pour injection parentérale (EPPI).

• **Caractérisation de la viscosité des gels G7a et REF7**

Les 2 gels G7a et REF7 sont de consistance visqueuse plus qu'élastique et sont donc caractérisés en viscosité.

La viscosité des gels stériles est caractérisée sur rhéomètre TA Instruments AR 2000 Ex, décrit en exemple 1, les résultats sont donnés dans le tableau IX ci-dessous.

Tableau IX

| | G7a | REF7 |
|---|---|---|
| Viscosité (Pa.s) | 67,1 | 35,4 |

La substitution permet d'obtenir des produits finis de rhéologie plus importante. Ces données rhéologiques, comme celles de l'exemple 2 (NaHA en conditions basiques), 6 (CMC en conditions basiques) et 7 (CH en conditions acides), confirment que la substitution peut se réaliser sur différents squelettes polysaccharidiques, et dans des conditions aussi bien acides que basiques.

**Exemple 8 : Substitution VSA (sel de sodium d'acide vinylsulfonique) sur NaHA simultanément à une réticulation par du DVS (divinylsulfone), à une température de 40°C et en milieu alcalin (RCR = 1,75:1)**

Cet exemple permet la mise en évidence par la rhéologie de la substitution sur un NaHA ponté avec différents réticulants.

• **Synthèse Gel G8**

Etape a) : Hydratation des fibres de hyaluronate de sodium sous forme de gel non réticulé

Des fibres de hyaluronate de sodium de qualité injectable ( 0,9 g soit 2,24 mmol; masse moléculaire : environ 2,7 MDa) ; sont pesées dans un récipient. Une solution aqueuse d'hydroxyde de sodium à 1 % (0,25 mol/L soit 3,92 mmol de $HO^-$ introduites dans le milieu) dans l'eau (15,7 g) est ajoutée (RCR = 1,75:1), le tout est homogénéisé pendant environ 1 heure à la spatule, à température ambiante et 900 mmHg.

Etape b) : Réticulation et Substitution

Du DVS (57 mg soit 0,48 mmol) et du VSA (87 mg soit 0,67 mmol) sont ajoutés au gel de hyaluronate de sodium (NaHA) non réticulé obtenu à l'étape précédente, le tout étant homogénéisé à la spatule pendant environ 30 minutes à température 12-14°C. L'ensemble est ensuite placé au bain-marie à 40°C pendant 1h00. Le degré de réticulant introduit DRI est égal à environ 0,21 et le degré de substituant introduit DSI est égal à environ 0,30.

Etape c) : Neutralisation, purification

Le gel final réticulé et substitué est ensuite neutralisé par l'ajout d'HCl 1N, et placé dans un bain de tampon

phosphate pour stabiliser le pH et permettre son hydratation, ou gonflement pour obtenir un gel comprenant 20 mg/g de HA, ce gel est ensuite homogénéisé avant d'être conditionné en seringues. On obtient ainsi un hydrogel G8 de NaHA réticulé et substitué.

• **Synthèse Gel REF8**

Le gel REF8 est synthétisé suivant le mode opératoire du gel G8 décrit ci-dessus, en remplaçant le VSA par de l'eau pour injection parentérale (EPPI).

• **Caractérisation de l'élasticité des gels G8 et REF8**

L'élasticité des gels est caractérisée sur rhéomètre TA Instruments AR 2000 Ex, décrit en exemple 2, les résultats sont donnés dans le tableau X ci-dessous.

Tableau X

| | G8 | REF8 |
|---|---|---|
| Elasticité : G' (Pa) à 1 Hz | 110 | 103 |

La substitution permet d'obtenir des produits finis dont l'élasticité est plus importante. Ces données rhéologiques, comme celles de l'exemple 2 (réticulation avec BDDE), confirment que la substitution peut se réaliser en même temps qu'un pontage du polysaccharide, quelle que soit la nature du réticulant.

**Exemple 9** :

**Substitution ASS (allylsulfate de sodium) sur NaHA simultanément à une réticulation par du BDDE (éther de 1,4-butanedioldiglycidyle), à une température de 50°C et en milieu alcalin (RCR = 0,8:1)**

Cet exemple permet la mise en évidence par la rhéologie :

- de la substitution de la fonction allyle sur le NaHA en cours de réticulation et de la différence de structure apportée par la substitution,
- de la meilleure résistance à la dégradation radicalaire du gel substitué avec un groupement pendant sulfate.

• **Synthèse Gel G9**

Etape a) : Identique à l'étape a) de synthèse du gel G1A.

Etape b) : Réticulation et Substitution

Du BDDE (65 mg soit 0,32 mmol) et du ASS (111 mg soit 0,68 mmol) sont ajoutés au gel de hyaluronate de sodium (NaHA) non réticulé obtenu à l'étape précédente, le tout étant homogénéisé à la spatule pendant environ 30 minutes à température 12-14°C. L'ensemble est ensuite placé au bain-marie à 50°C pendant 2h10. Le degré de réticulant introduit DRI est égal à environ 0,14 et le degré de substituant introduit DSI est égal à environ 0,30.

Etape c) : Neutralisation, purification

Le gel final réticulé et substitué est ensuite neutralisé par l'ajout d'HCl 1N, et placé dans un bain de tampon phosphate pour stabiliser le pH et permettre son hydratation, ou gonflement pour obtenir un gel comprenant 20 mg/g de HA, ce gel est ensuite homogénéisé avant d'être conditionné en seringues qui sont stérilisées par autoclavage. On obtient ainsi un hydrogel G9 de NaHA réticulé, substitué et stérilisé.

• **Synthèse Gel REF9**

Le gel REF9 est synthétisé suivant le mode opératoire du gel G9 décrit ci-dessus, en remplaçant l'ASS par de l'eau pour injection parentérale (EPPI).

• **Caractérisation de l'élasticité des gels G9 et REF9**

L'élasticité des gels stériles est caractérisée sur rhéomètre TA Instruments AR 2000 Ex, décrit en exemple 2, les résultats sont donnés dans le tableau XI ci-dessous.

Tableau XI

| | G9 | REF9 |
|---|---|---|
| Elasticité : G' (Pa) à 1 Hz | 123 | 105 |

La substitution permet d'obtenir des produits finis de rhéologie plus importante (+17 %). Ces données rhéologiques confirment la substitution de la fonction allyle sur le NaHA simultanément à la réticulation.

• **Test de dégradation radicalaire des gels G9 et REF9**

Les gels ont également été caractérisés par un test de dégradation radicalaire *in vitro* à la température, décrit en exemple 2. Les temps de demi-vie obtenus sont donnés dans le tableau XII ci-dessous.

Tableau XII

| | G9 | REF9 |
|---|---|---|
| Temps de demi-vie (minutes) | 12,6 | 6,6 |

Ainsi, les temps de demi-vie des gels substitués avec un groupement pendant sulfate obtenus selon l'invention sont plus longs, garantissant un temps de rémanence *in vivo* supérieur.

**Contre-exemples**

**Contre-Exemple 1** :

Ce contre-exemple est similaire à l'exemple 1 réalisé dans des conditions de température et de pH drastiques de l'art antérieur.

**a) Synthèse Gel G1Ca: Substitution du VSA (sel de sodium d'acide vinylsulfonique) sur NaHA en simultanément à une réticulation par du BDDE (éther de 1,4-butanedioldiglycidyle), à une température de 80°C et en milieu alcalin concentré (RCR de 4,1:1)**

Etape a) : Hydratation des fibres de hyaluronate de sodium sous forme de gel non réticulé

Des fibres de hyaluronate de sodium de qualité injectable (0,9 g soit 2,24 mmol ; masse moléculaire : environ 2,7 MDa) ; sont pesées dans un récipient. Une solution aqueuse d'hydroxyde de sodium à 5 % (1,25 mol/L soit 9,25 mmol de $HO^-$ introduites dans le milieu) dans l'eau (7,4 g) est ajoutée (RCR de 4,1:1), le tout est homogénéisé pendant environ 1 heure à la spatule, à température ambiante et 900 mmHg.

Etape b) : Réticulation et Substitution

Du BDDE (65 mg soit 0,32 mmol) et du VSA (102 mg soit 0.78 mmol) sont ajoutés au gel de hyaluronate de sodium (NaHA) non réticulé obtenu à l'étape précédente, le tout étant homogénéisé à la spatule pendant environ 30 minutes à température 12-14°C. L'ensemble est ensuite placé au bain-marie à 80°C pendant 2h10 afin d'obtenir un degré de réticulant introduit DRI d'environ 0,14 et un degré de substituant introduit DSI d'environ 0,35.

Au bout de 20 minutes à 80°C, le milieu réactionnel est complètement dégradé (liquide, marron, aspect « caramélisé »).

Une température élevée (80°C) et l'utilisation d'une soude concentrée dégradent le réseau polysaccharidique et gênent ainsi la substitution et la réticulation des chaines de polymère.

**b) Synthèse Gel G1Cb: Substitution du VSA (sel de sodium d'acide vinylsulfonique) sur NaHA en simultanément à une réticulation par du BDDE (éther de 1,4-butanedioldiglycidyle), à une température de 80°C et en milieu alcalin concentré (RCR de 8,2:1)**

Etape a) : Hydratation des fibres de hyaluronate de sodium sous forme de gel non réticulé

Des fibres de hyaluronate de sodium de qualité injectable (0,9 g soit 2,24 mmol ; masse moléculaire : environ 2,7 MDa) ; sont pesées dans un récipient. Une solution aqueuse d'hydroxyde de sodium à 10 % (2,5 mol/L soit 18,5 mmol de $HO^-$ introduites dans le milieu) dans l'eau (7,4 g) est ajoutée (RCR de 8,2: 1), le tout est homogénéisé pendant environ 1 heure à la spatule, à température ambiante et 900 mmHg.

Etape b) : Réticulation et Substitution

Du BDDE (65 mg soit 0,32 mmol) et du VSA (102 mg soit 0,78 mmol) sont ajoutés au gel de hyaluronate de sodium (NaHA) non réticulé obtenu à l'étape précédente, le tout étant homogénéisé à la spatule pendant environ 30 minutes à température 12-14°C. L'ensemble est ensuite placé au bain-marie à 80°C pendant 2h10 afin d'obtenir un degré de réticulant introduit DRI d'environ 0,14 et un degré de substituant introduit DSI d'environ 0,35.

Au bout de 15 minutes à 80°C, le milieu réactionnel est complètement dégradé (liquide, marron, aspect « caramélisé »).

Une température élevée (80°C) et l'utilisation d'une soude concentrée dégradent le réseau polysaccharidique et gênent ainsi la substitution et la réticulation des chaines de polymère.

**Contre-Exemple 2 (contre-exemple non représentatif de l'invention)** :

Ce contre-exemple est similaire à l'exemple 6 réalisé dans des conditions de température et de pH drastiques de l'art antérieur.

**a) Substitution VSA (sel de sodium d'acide vinylsulfonique) sur CMC (carboxyméthylcellulose) simultanément à une réticulation par du BDDE (éther de 1,4-butanedioldiglycidyle) à une température de 80°C et en milieu alcalin concentré (RCR de 5,1:1)**

• **Synthèse Gel G6a**

Etape a) : Hydratation de la CMC sous forme de gel non réticulé

0,93 g soit 2,20 mmol de CMC de sodium (fournie par Sigma, masse moléculaire : environ 1,0 MDa) est pesée dans un récipient. Une solution aqueuse d'hydroxyde de sodium à 5 % (1,25 mol/L soit 11,25 mmol de $HO^-$ introduites dans le milieu) dans l'eau (9,0 g) est ajoutée (RCR de 5,1:1), le tout est homogénéisé pendant environ 90 minutes à la spatule, à température ambiante et 900 mmHg.

Etape b) : Réticulation et Substitution

Du BDDE (37 mg soit 0,18 mmol) et du VSA (87 mg soit 0,67 mmol) sont ajoutés au gel de CMC non réticulé obtenu à l'étape précédente, le tout étant homogénéisé à la spatule pendant environ 30 minutes à température 12-14°C. L'ensemble est ensuite placé au bain-marie à 80°C pendant 3h35 afin d'obtenir un degré de réticulant introduit DRI d'environ 0,08 et un degré de substituant introduit DSI d'environ 0,30.

Etape c) : Neutralisation, purification

Le gel final réticulé et substitué est ensuite neutralisé par l'ajout d'HCl 1N, et placé dans un bain de tampon phosphate pour stabiliser le pH et permettre son hydratation, ou gonflement pour obtenir un gel comprenant 30 mg/g de CMC, ce gel est ensuite homogénéisé avant d'être conditionné en seringues. On obtient ainsi un hydrogel G6a de CMC réticulé et substitué.

Le gel a un aspect trop liquide, trop dispersif, pas assez élastique.

**b) Substitution VSA (sel de sodium d'acide vinylsulfonique) sur CMC (carboxyméthylcellulose) simultanément à une réticulation par du BDDE (éther de 1,4-butanedioldiglycidyle) à une température de 80°C et en milieu alcalin concentré (RCR de 10.2:1)**

• **Synthèse Gel G6b**

Etape a) : Hydratation de la CMC sous forme de gel non réticulé

0,93 g soit 2,20 mmol de CMC de sodium (fournie par Sigma, masse moléculaire : environ 1,0 MDa) est pesée dans un récipient. Une solution aqueuse d'hydroxyde de sodium à 10 % (2,5 mol/L soit 22,5 mmol de $HO^-$ introduites dans le milieu) dans l'eau (9,0 g) est ajoutée (RCR de 10,2:1), le tout est homogénéisé pendant environ 90 minutes à la spatule, à température ambiante et 900 mmHg.

Etape b) : Réticulation et Substitution

Du BDDE (37 mg soit 0,18 mmol) et du VSA (87 mg soit 0,67 mmol) sont ajoutés au gel de CMC non réticulé obtenu à l'étape précédente, le tout étant homogénéisé à la spatule pendant environ 30 minutes à température 12-14°C. L'ensemble est ensuite placé au bain-marie à 80°C pendant 3h35 afin d'obtenir un degré de réticulant introduit DRI d'environ 0,08 et un degré de substituant introduit DSI d'environ 0,30.

Etape c) : Neutralisation, purification

Le gel final réticulé et substitué est ensuite neutralisé par l'ajout d'HCl 1N, et placé dans un bain de tampon phosphate pour stabiliser le pH et permettre son hydratation, ou gonflement pour obtenir un gel comprenant 30 mg/g de CMC, ce gel est ensuite homogénéisé avant d'être conditionné en seringues. On obtient ainsi un hydrogel G6b de CMC réticulé et substitué.

Le gel a un aspect trop liquide, trop dispersif, pas assez élastique.

**c) Caractérisation de l'élasticité des gels G6a et G6b**

L'élasticité des gels est caractérisée sur rhéomètre TA Instruments AR 2000 Ex, décrit en exemple 2, les résultats sont donnés dans le tableau ci-dessous.

| | G6a | G6b | G6 | REF6 |
|---|---|---|---|---|
| Elasticité : G' (Pa) à 1 Hz | 17 | 12 | 524 | 483 |

Les données rhéologiques confirment les aspects observés : Les gels n'ont pas la consistance attendue, ils ne présentent pas les propriétés viscoélastiques exigées pour les applications visées.

Une température élevée (80°C) et l'utilisation d'une soude concentrée dégradent le réseau polysaccharidique et gênent ainsi le greffage et la réticulation des chaines de polymère.

## Revendications

1. Procédé de substitution et réticulation simultanées d'un polysaccharide via ses fonctions hydroxyles, en phase aqueuse, comprenant les étapes suivantes :

    - on dispose d'un polysaccharide en milieu aqueux, choisi dans le groupe constitué par l'acide hyaluronique ou l'un de ses sels, le chitosane et leurs dérivés.
    - on le met en présence d'au moins un précurseur d'un substituant, choisi dans le groupe constitué des molécules comprenant une seule fonction réactive choisie dans le groupe constitué des fonctions vinyle substituée ou non, époxyde substituée ou non, allyle substituée ou non, cétone, aldéhyde, thiocyanate, halide, isocyanate, halosilicium, nitrile, sultone.
    - on le met en présence d'un agent de réticulation,
    - on obtient et on isole le polysaccharide substitué et réticulé,

    **caractérisé en ce qu'**il est mis en oeuvre en présence d'un catalyseur, base ou acide, dont la concentration

est comprise entre $3,16.10^{-7}$ et 0,32 mol/L, et à une température inférieure à 60° C.

2. Procédé selon la revendication 1, **caractérisé en ce que** le ratio catalyseur réactionnel (RCR) défini comme étant :

$$RCR = \frac{\text{(Nombre de mole de fonctions réactives du catalyseur introduites dans le milieu réactionnel)}}{\text{(Nombre de moles de motif disaccharidique introduites dans le milieu réactionnel)}}$$

est compris entre 0,02: 1 et 3:1.

3. Procédé selon l'une quelconque des revendications 1 à 2, **caractérisé en ce que** le catalyseur est une base.

4. Procédé selon la revendication 3, **caractérisé en ce que** la base est une base inorganique choisie parmi la soude ou la potasse et que et la fonction réactive du catalyseur est l'ion $OH^-$.

5. Procédé selon la revendication précédente, **caractérisé en ce que** la concentration massique de la base inorganique est comprise entre 1,2.10-5 et 1,15 %.

6. Procédé selon l'une quelconque des revendications 3 à 5, **caractérisé en ce que** la concentration en catalyseur $HO^-$ est comprise entre $10^{-6}$ mol/L et 0,32 mol/L, tel que $10^{-6}$ mol/L $\leq [HO^-] \leq$ 0,32 mol/L.

7. Procédé selon l'une quelconque des revendications 3 à 6, **caractérisé en ce que** le pH du milieu réactionnel aqueux est basique et est compris entre 8,5 et 13,5.

8. Procédé selon la revendication 1, **caractérisé en ce que** la masse moléculaire Mw du polysaccharide est comprise dans un intervalle de 0,01 MDa à 4,0 MDa.

9. Procédé selon l'une quelconque des revendications précédentes, **caractérisé en ce que** l'agent de réticulation est bi- ou polyfonctionnel.

10. Procédé selon la revendication 9, **caractérisé en ce que** l'agent de réticulation bi-ou polyfonctionnel est choisi parmi le groupe constitué par l'éther d'éthylèneglycoldiglycidyle l'éther de butanedioldiglycidyle, l'éther de polyglycérolpolyglycidyle, l'éther de polyéthylèneglycoldiglycidyle, l'éther de polypropylèneglycoldiglycidyle, un bis- ou polyépoxy tel que 1,2,3,4-diépoxybutane ou 1,2,7,8-diépoxyoctane, une dialkylsulfone, la divinylsulfone, le formaldéhyde ou bien encore le glutaraldéhyde.

11. Procédé selon la revendication 1, **caractérisé en ce que** le précurseur du substituant est choisi dans le groupe constitué des molécules comportant en outre au moins une fonction ou un groupement d'intérêt, choisi dans le groupe constitué par les groupements ou fonctions sulfonate, alkyle linéaire ou ramifié, aromatique substitué ou non, sulfate, thiol, ose , phosphate, phosphonate, carbonate et ester.

12. Procédé selon l'une quelconque des revendications précédentes, **caractérisé en ce que** le polysaccharide obtenu présente un degré de substituant introduit défini comme étant :

$$DSI = \frac{\text{(Nombre de moles de fonctions réactives du substituant introduites dans le milieu réactionnel)}}{\text{(Nombre de moles de motif disaccharidique introduites dans le milieu réactionnel}}$$

compris dans l'intervalle allant de 0,001 à 4,00.

13. Procédé selon l'une quelconque des revendications précédentes, **caractérisé en ce que** le polysaccharide obtenu présente un taux de réticulant introduit DRI défini comme étant :

$$\text{DRI} = \frac{\text{(Nombre de moles de réticulant introduites dans le milieu réactionnel)}}{\text{(Nombre de moles de motif disaccharidique introduites dans le milieu réactionnel)}}$$

compris dans l'intervalle allant de 0,001 à 0,5.

**14.** Procédé selon l'une quelconque des revendications précédentes, **caractérisé en ce qu'**il est mis en oeuvre à une température comprise entre 39 C et 60° C.

**15.** Procédé selon l'une des revendications précédentes, **caractérisé en ce qu'**il est mis en oeuvre pendant une durée comprise dans l'intervalle allant de 15 minutes à 48 heures.

**16.** Procédé selon l'une des revendications précédentes, **caractérisé en ce qu'**il comprend en outre une étape de lavage du polysaccharide obtenu.

**17.** Composition comprenant un polysaccharide obtenu par le procédé selon l'une quelconque des revendications précédentes.

**18.** Utilisation d'un polysaccharide obtenu selon le procédé selon l'une des revendications 1 à 16, pour la formulation d'une composition de viscosupplémentation.

**19.** Utilisation d'un polysaccharide obtenu selon le procédé selon l'une des revendications 1 à 16, pour la formulation d'une composition pour le comblement des rides.

**20.** Kit comprenant un polysaccharide obtenu selon le procédé selon l'une des revendications 1 à 16, conditionné en seringue stérile.

**Patentansprüche**

**1.** Verfahren zum gleichzeitigen Substituieren und Vernetzen eines Polysaccharides vermittels seiner funktionellen Hydroxylgruppen, in einer wässrigen Phase, umfassend die folgenden Schritte:

- Bereitstellen eines Polysaccharides in einem wässrigen Medium, das ausgewählt ist aus der Gruppe bestehend aus Hyaluronsäure oder einem seiner Salze, Chitosan und deren Derivaten,
- in Kontakt bringen desselben mit wenigstens einem Vorläufer eines Substituenten, der ausgewählt ist aus der Gruppe bestehend aus Molekülen umfassend eine einzelne reaktive Gruppe, die ausgewählt ist aus der Gruppe bestehend aus substituiertem oder nicht-substituiertem Vinyl, substituiertem oder nicht-substituiertem Epoxid, substituiertem oder nicht-substituiertem Allyl, Keton, Aldehyd, Thiocyanat, Halid, Isocyanat, Halosilizium, Nitril und Sulton,
- in Kontakt bringen desselben mit einem Vernetzungsmittel,
- Erhalten und Isolieren des substituierten und vernetzten Polysaccarids,

**dadurch gekennzeichnet, dass** das Verfahren durchgeführt wird in Gegenwart eines basischen oder eines sauren Katalysators, dessen Konzentration zwischen $3{,}16 \times 10^{-7}$ und 0,32 mol/l beträgt, und bei einer Temperatur von weniger als 60 °C.

**2.** Verfahren nach Anspruch 1, **dadurch gekennzeichnet, dass** das reaktive Katalysatorverhältnis (RCR), das wie folgt definiert ist:

RCR = (in das Reaktionsmedium eingeführte Molanzahl an reaktiven funktionellen Gruppen des Katalysators)/(in das Reaktionsmedium eingeführte Molanzahl an Disaccharideinheiten),

zwischen 0,02: 1 und 3: 1 beträgt.

3. Verfahren nach einem der Ansprüche 1 bis 2, **dadurch gekennzeichnet, dass** der Katalysator eine Base ist.

4. Verfahren nach Anspruch 3, **dadurch gekennzeichnet, dass** die Base eine anorganische Base ist, die ausgewählt ist aus Natriumhydroxid oder Kaliumhydroxid, und dass die reaktive funktionelle Gruppe des Katalysators das $OH^-$- Ion ist.

5. Verfahren nach einem der vorangehenden Ansprüche, **dadurch gekennzeichnet, dass** die Massenkonzentration der anorganischen Base zwischen $1,2\times10^{-5}$ und 1,15% beträgt.

6. Verfahren nach einem der Ansprüche 3 bis 5, **dadurch gekennzeichnet, dass** die Konzentration an Katalysator-$OH^-$ zwischen $10^{-6}$ mol/l und 0,32 mol/l beträgt, so dass $10^{-6}$ mol/l $\leq [OH^-] \leq$ 0,32 mol/l.

7. Verfahren nach einem der Ansprüche 3 bis 6, **dadurch gekennzeichnet, dass** der pH des wässrigen Reaktionsmediums basisch ist und zwischen 8,5 und 13,5 beträgt.

8. Verfahren nach Anspruch 1, **dadurch gekennzeichnet, dass** das Molekulargewicht Mw des Polysaccharides in einem Bereich von 0,01 MDa bis 4,0 MDa liegt.

9. Verfahren nach einem der vorangehenden Ansprüche, **dadurch gekennzeichnet, dass** das Vernetzungsmittel bi- oder polyfunktionell ist.

10. Verfahren nach Anspruch 9, **dadurch gekennzeichnet, dass** das bi- oder polyfunktionelle Vernetzungsmittel ausgewählt ist aus der Gruppe bestehend aus Ethylenglycoldiglycidylether, Butandioldiglycidylether, Polyglycerolpolyglycidylether, Polyethylenglycoldiglycidylether, Polypropylenglycoldiglycidylether, einem Bis- oder Polyepoxid wie 1,2,3,4-Diepoxybutan oder 1,2,7,8-Diepoxyoktan, einem Dialkylsulfon, Divinylsulfon, Formaldehyd oder Glutaraldehyde.

11. Verfahren nach Anspruch 1, **dadurch gekennzeichnet, dass** der Vorläufer des Substituenten ausgewählt ist aus der Gruppe bestehend aus Molekülen, die ausserdem mindestens eine funktionelle Gruppe oder einen vorteilhaften Rest umfassen, die/der ausgewählt ist aus der Gruppe bestehend aus den funktionellen Gruppen oder Resten Sulfonat, linearem oder verzweigtem Alkyl, substituiertem oder nicht-substituiertem Aromaten, Sulfat, Thiol, Os, Phosphat, Phosponat, Carbonat und Ester.

12. Verfahren nach einem der vorangehenden Ansprüche, **dadurch gekennzeichnet, dass** das erhaltene Polysaccharid einen Grad an eingeführtem Substituenten aufweist, der wie folgt definiert ist:

DSI = (in das Reaktionsmedium eingeführte Molanzahl an reaktiven funktionellen Gruppen des Substituenten)/(in das Reaktionsmedium eingeführte Molanzahl an Disaccharideinheiten)

und enthalten ist in einem Bereich, der sich von 0,001 bis 4,00 erstreckt.

13. Verfahren nach einem der vorangehenden Ansprüche, **dadurch gekennzeichnet, dass** das erhaltene Polysaccharid einen Grad an eingeführter Vernetzung DRI aufweist, der wie folgt definiert ist

DRI = (in das Reaktionsmedium eingführte Molanzahl an Vernetzungsmittel)/(in das Reaktionsmedium eingeführte Molanzahl an Dissacharideinheiten)

und enthalten ist in einem Bereich, der sich von 0,001 bis 0,5 erstreckt.

14. Verfahren nach einem der vorangeheneden Ansprüche, **dadurch gekennzeichnet, dass** es bei einer Temperatur zwischen 39°C und 60°C durchgeführt wird.

15. Verfahren nach einem der vorangehenden Ansprüche, **dadurch gekennzeichnet, dass** es für die Dauer im Bereich von 15 Minuten bis 48 Stunden durchgeführt wird.

**16.** Verfahren nach einem der vorangehenden Ansprüche, **dadurch gekennzeichnet, dass** es außerdem einen Waschschritt des erhaltenen Polysaccharids umfasst.

**17.** Zusammensetzung umfassend ein durch ein Verfahren nach einem der vorangehenden Ansprüche erhaltenes Polysaccharid.

**18.** Verwendung eines gemäß einem Verfahren nach einem der Ansprüche 1 bis 16 erhaltenen Polysaccharids für die Formulierung einer Zusammensetzung zur Viskosupplementation.

**19.** Verwendung eines gemäß einem Verfahren nach einem der Ansprüche 1 bis 16 erhaltenen Polysaccharids für die Formulierung einer Zusammensetzung zur Faltenauffüllung.

**20.** Kit umfassend ein gemäß einem Verfahren nach einem der Ansprüche 1 bis 16 erhaltenes Polysaccharid, in einer sterilen Spritze verpackt.

## Claims

**1.** A process for performing simultaneous substitution and crosslinking of a polysaccharide *via* its hydroxyl functional groups, in an aqueous reaction medium, comprising the following steps:

- A polysaccharide is placed in an aqueous reaction medium, the polysaccharide is chosen from the group consisting of hyaluronic acid and salts thereof, chitosan, cellulose, and derivatives thereof;
- It is brought into the presence of at least one precursor of the substituent, the precursor is chosen from the group consisting of compounds comprising a single reactive functional group being chosen from the group consisting of substituted or unsubstituted vinyl, substituted or unsubstituted epoxide, substituted or unsubstituted allyl, ketone, aldehyde, thiocyanate, halide, isocyanate, halosilicon, nitrile and sultone functional groups;
- It is brought into the presence of a crosslinking agent ;
- The substituted and crosslinked polysaccharide is obtained and isolated

**characterized in that** the process is performed in the presence of a basic or acidic catalyst at a concentration between $3.16 \times 10^{-7}$ and 0.32 mol/L, and at a temperature of less than 60°C.

**2.** Process according to claim 1, **characterized in that** reactive catalyst ratio (RCR) is defined as being:

$$RCR = \frac{\text{(Number of moles reactive functional groups of the catalyst introduced into the reaction medium)}}{\text{(Number of moles disacharide unit introduced into the reaction medium)}}$$

is between 0.02:1 and 3:1.

**3.** Process according to any one of the claims 1 to 2, **characterized in that** the catalyst is a base.

**4.** Process according to claim 3, **characterized in that** the base is an inorganic base chosen from sodium hydroxide or potassium hydroxide and wherein the reactive functional group of the catalyst is the $OH^-$ ion.

**5.** Process according to the preceding claim, **characterized in that** the concentration by weight of the inorganic base is between $1.2 \times 10^{-5}$ % and 1.15 %.

**6.** The process according to any one of the claims 3 to 5, **characterized in that** the concentration of the catalyst $OH^-$ ion is $10^{-6}$ mol/L and 0.32 mol/L, such that $10^{-6}$ mol/L $\leq [HO^-] \leq$ 0.32 mol/L.

**7.** Process according to any one of the claims 3 to 6, **characterized in that** the pH of the aqueous reaction medium is basic and is between 8.5 and 13.5.

**8.** Process according to claim 1, **characterized in that** the molecular weight Mw of the polysaccharide is within a range from 0.01 MDa to 4.0 MDa.

9. Process according to any one of the preceding claims, **characterized in that** the the crosslinking agent is bi- or polyfunctional.

10. Process according to claim 9, **characterized in that** the bi- or polyfunctional crosslinking agent is chosen from the group consisting of ethylene glycol diglycidyl ether, butanediol diglycidyl ether, polyglycerol polyglycidyl ether, polyethylene glycol diglycidyl ether, polypropylene glycol diglycidyl ether, a bisepoxy or a polyepoxy such as 1,2,3,4-diepoxybutane or 1,2,7,8-diépoxyoctane, a dialkyl sulfone, divinyl sulfone, formaldehyde, and glutaraldehyde.

11. Process according to claim 1, **characterized in that** the precursor of the substituent is chosen from the group consisting of molecules additionally comprising at least one advantageous functional group or group chosen from the group consisting of sulfonate, linear or branched alkyl, substituted or unsubstituted aromatic, sulfate, thiol, monosaccharide, phosphate, phosphonate, carbonate and ester groups or functional groups.

12. Process according to any one of the preceding claims, **characterized in that** the obtained polysaccharide has a degree of substituent introduced (DSI), defined as being:

$$DSI = \frac{\text{(Number of moles of reactive functional groups of the substituent introduced into the reaction medium)}}{\text{(Number of moles of disaccharide unit introduced into the reaction medium)}}$$

within a range from 0.001 to 4.00.

13. Process according to any one of the preceding claims, **characterized in that** the obtained polysaccharide has a degree of crosslinking agent introduced (DCI), defined as being:

$$DCI = \frac{\text{(Number of moles crosslinking agent introduced into the reaction medium)}}{\text{(Number of moles disacharide unit introduced into the reaction medium)}}$$

within a range from 0.001 to 0.5.

14. Process according to any one of the preceding claims, **characterized in that** it is carried out at temperature between 39 °C and 60 °C.

15. Process according to any one of the preceding claims, **characterized in that** it is carried out for a time period from 15 minutes to 48 hours.

16. Process according to any one of the preceding claims, **characterized in that** it additionally comprises a step of washing the polysaccharide obtained.

17. A composition comprising a polysaccharide obtained by the process according to any one of the preceding claims.

18. Use of a polysaccharide obtained by the process according to any one of the claims 1 to 16, to formulate a viscosupplementation composition.

19. Use of a polysaccharide obtained by the process according to any one of the claims 1 to 16, to formulate a composition for filling in wrinkles.

20. A kit comprising a polysaccharide obtained by to the process according to any one of the claims 1 to 16, packaged in a sterile syringe.

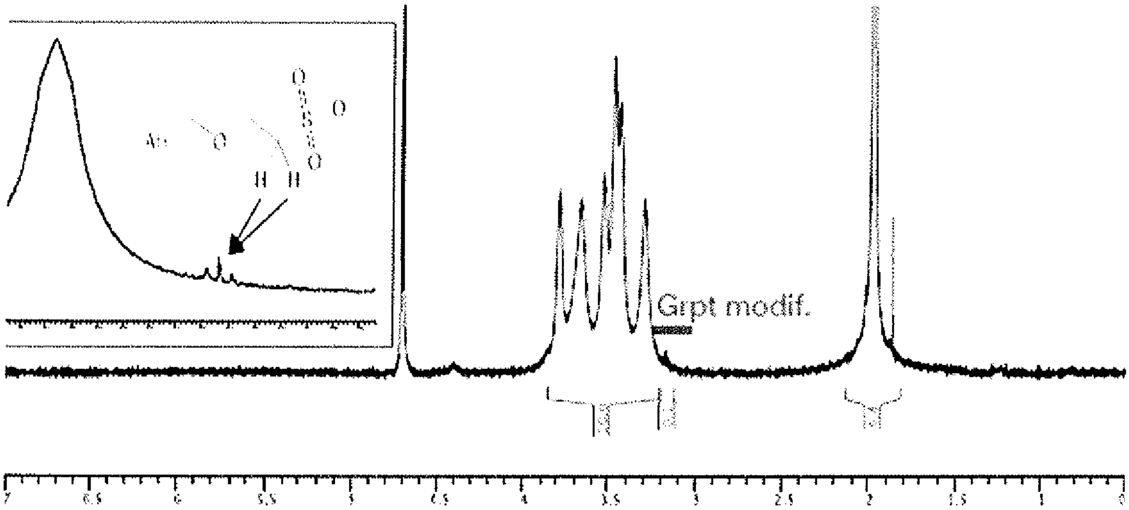


Figure 1

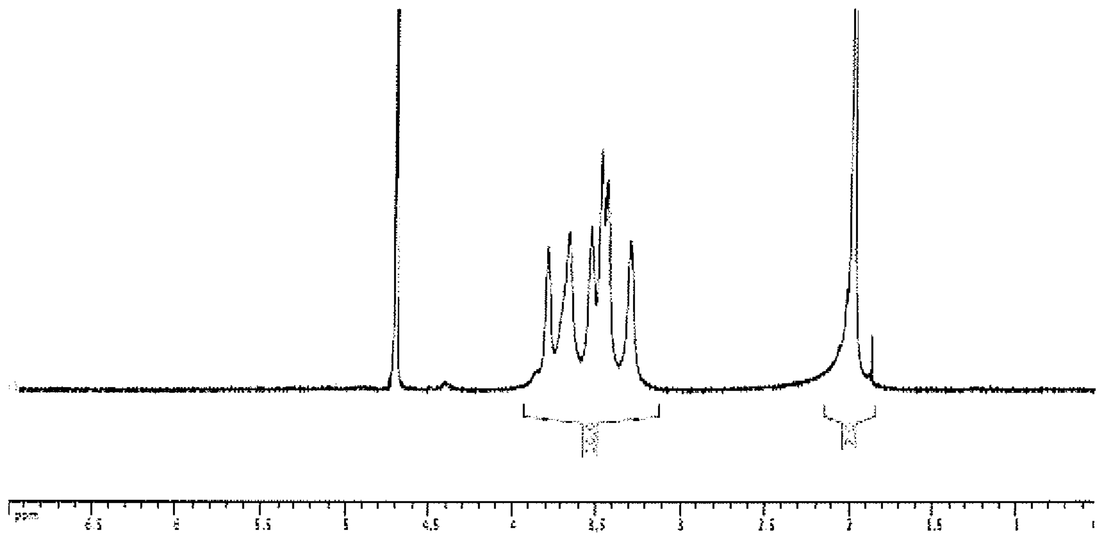

Figure 2

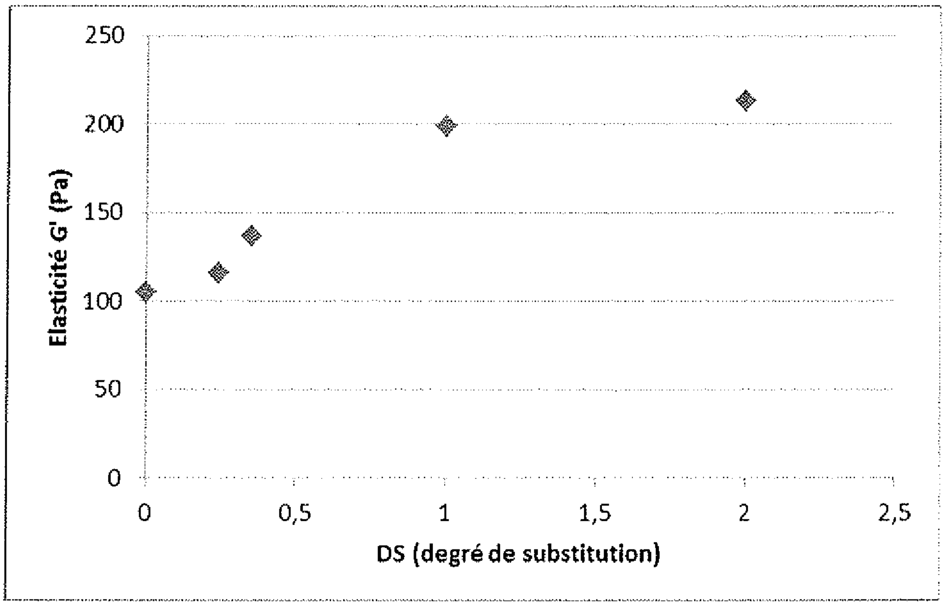
250
200
150
100
50
0
0
0,5
1
1,5
2
2,5
Elasticité G' (Pa)
DS (degré de substitution)

Figure 3

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 0256116 A
- EP 0341745 A
- WO 9943728 A
- EP 0749982 A, HERCULES
- US 4716224 A
- US 4863907 A
- EP 0507604 A2
- US 4716154 A
- US 4772419 A
- US 4957744 A
- US 4582865 A
- US 4605691 A
- US 5128326 A
- US 4713448 A
- WO 2005012364 A, ANTEIS
- US 4990609 A, WOLFF WALSRODE
- US 3046272 A
- US 4321367 A
- US 4175183 A


### Littérature non-brevet citée dans la description


- **SIMKOVIC et al.** *Carbohydrate Polymers,* 2000, vol. 41, 9-14
- **ROCHA DE SOUZA.** Antioxidant activities of sulfated polysaccharides from brown and red seaweeds. *J. Appl. Phycol.,* 2007, vol. 19, 153-160